(19) Europäisches Patentamt
European Patent Office
Office européen des brevets

(11) EP 1 758 860 B1

(12) **EUROPÄISCHE PATENTSCHRIFT**

(45) Veröffentlichungstag und Bekanntmachung des Hinweises auf die Patenterteilung:
**02.09.2009 Patentblatt 2009/36**

(21) Anmeldenummer: **05766092.0**

(22) Anmeldetag: **20.06.2005**

(51) Int Cl.:
***C07D 213/38*** (2006.01) ***C07D 417/06*** (2006.01)
***C07D 413/06*** (2006.01) ***A61K 31/435*** (2006.01)
***A61P 25/00*** (2006.01)

(86) Internationale Anmeldenummer:
**PCT/EP2005/006624**

(87) Internationale Veröffentlichungsnummer:
**WO 2005/123681 (29.12.2005 Gazette 2005/52)**

(54) **GESÄTTIGTE UND UNGESÄTTIGTE 3-PYRIDYL-BENZOCYCLOALKYLMETHYL-AMINE ZUR BEHANDLUNG VON SCHMERZEN, DEPRESSIONEN UND ANGSTZUSTÄNDEN**

SATURATED AND UNSATURATED 3-PYRIDYL-BENZOCYCLOALKYLMETHYL-AMINES FOR USE IN THE TREATMENT OF PAINS, DEPRESSIONS AND ANXIETY STATES

3-PYRIDYL-BENZOCYCLOALKYLMETHYLAMINES SATUREES ET INSATUREES DESTINEES AU TRAITEMENT DE DOULEURS, DE DEPRESSIONS ET D'ETATS D'ANGOISSE

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**HR LV**

(30) Priorität: **22.06.2004 DE 102004030099**

(43) Veröffentlichungstag der Anmeldung:
**07.03.2007 Patentblatt 2007/10**

(73) Patentinhaber: **Grünenthal GmbH**
**52078 Aachen (DE)**

(72) Erfinder:
- **SUNDERMANN, Bernd**
**52074 Aachen (DE)**
- **HENNIES, Hagen-Heinrich**
**52152 Simmerath (DE)**
- **SCHIENE, Klaus**
**40227 Düsseldorf (DE)**
- **BLOMS-FUNKE, Petra**
**52146 Würselen (DE)**
- **ENGLBERGER, Werner**
**52223 Stolberg (DE)**
- **FRORMANN, Sven**
**52066 Aachen (DE)**
- **SAUNDERS, Derek**
**52072 Aachen (DE)**

(74) Vertreter: **Brosch, Oliver et al**
**Kutzenberger & Wolff**
**Theodor-Heuss-Ring 23**
**50668 Köln (DE)**

(56) Entgegenhaltungen:
**WO-A-99/58490** **US-A- 4 845 221**

- **PATENT ABSTRACTS OF JAPAN Bd. 2003, Nr. 12, 5. Dezember 2003 (2003-12-05) & JP 2004 149429 A (TAKEDA CHEM IND LTD), 27. Mai 2004 (2004-05-27)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 758 860 B1

## Beschreibung

Die vorliegende Erfindung betrifft gesättigte und ungesättigte 3-Pyridyl-benzocycloalkylmethyl-amine, Verfahren zu deren Herstellung, Arzneimittel enthaltend diese Verbindungen und die Verwendung von gesättigten und ungesättigten 3-Pyridyl-benzocycloalkylmethyl-amine zur Herstellung von Arzneimitteln sowie Verfahren zur Behandlung von Schmerzen, Depressionen und/oder Angststörungen.

Die Behandlung akuter und chronischer Schmerzzustände hat in der Medizin eine große Bedeutung. Es besteht ein weltweiter Bedarf an gut wirksamen Schmerztherapien für eine patientengerechte und zielorientierte Behandlung akuter und chronischer Schmerzen, wobei hierunter die erfolgreiche und zufrieden stellende Schmerzbehandlung für den Patienten zu verstehen ist. Dies zeigt sich in der großen Anzahl von wissenschaftlichen Arbeiten, die auf dem Gebiet der angewandten Analgetik bzw. Grundlagenforschung zur Nociception in der letzten Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Mit dem Ziel einer verbesserten Behandlung werden in der Klinik häufig Kombinationen von Opiaten mit Hemmern der Wiederaufnahme der Monoamine Serotonin (5-HT) und/oder Noradrenalin (NA) bei chronischen Schmerzen (u.a. Entzündungsschmerz, Tumorschmerz) eingesetzt (Sindrup, in: Yaksh, T.L., et al., Anesthesia. Biological foundations. Philadelphia: Lippincott-Raven, 1997, 987-997). Da darüber hinaus chronische Schmerzen bei einer Vielzahl von Patienten mit Angststörungen oder Depressionen vergesellschaftet sind, ist eine Substanz mit μ-opiatrezepotoragonistischen Eigenschaften kombiniert mit einer klinisch relevanten Serotonin- und / oder Noradrenalin-Wiederaufnahmehemmung besonders günstig.

Darüber hinaus werden Wiederaufnahmehemmer von Noradrenalin und Serotonin klinisch auch zur Monotherapie bei chronischen Schmerzzuständen eingesetzt (Sindrup, in: Yaksh, T.L., et al., Anesthesia. Biological foundations. Philadelphia: Lippincott-Raven, 1997, 987-997). Monoaminwiederaufnahmehemmer verfügen über eine eigenständige analgetische Wirkung, indem absteigende Schmerzhemmbahnen auf der Ebene des Rückenmarks aktiviert werden.

Die Verwendung der Monoaminwiederaufnahmehemmer ist durch Nebenwirkungen wie z.B. Akkomodationsstörungen, Serotoninsyndrom oder QT-Verlängerungen eingeschränkt. Es besteht nach wie vor ein dringender Bedarf zur patientengerechten Behandlung insbesondere chronischer Schmerzen.

Eine breite klinische Verwendung finden Wiederaufnahmehemmer von Noradrenalin und Serotonin in der Behandlung von Depressionen und Angststörungen (Pacher, P., Kohegyi, E., Kecskemeti, V., Furst, S., Current Medicinal Chemistry 2001, 8, 89-100; Goddard, A. W., Coplan, J.D., Gorman, J. M., Chamey, D. S., In: Neurobiology of mental illness, Charney, D.S., Nestler, E.J., Bunney, B.S. (Hrsg.), Oxford University Press, New York, 1999, S. 548-563).

Depressionen sind Störungen der Affektivität, bei der ein depressives Syndrom im Vordergrund steht, wobei depressiv mit Verstimmung verbunden bzw. traurig gestimmt heißt. Zu den depressiven Erkrankungen werden unipolare schwere Depressionen mit oder ohne Wahn, mittelgradige Depressionen, leichte Depressionen, Dysthymie, Melancholie, bipolare Depressionen (Bipolare Erkrankungen I, Manie und schwere Depression; Bipolare Erkrankungen II, Hypomanie und schwere Depressionen; zykiothyme Persönlichkeitsstörungen, Hypomanie) und milde Depressionen gezählt. Angststörungen werden in Generalisierte Angststörungen, Panikattacken, Zwangssyndrome (englisch: obessessive compulsive disorders, OCD), Soziale Angststörungen, Einfache Phoblen, Agoraphobien, Posttraumatische Belastungsstörungen (englisch posttraumatic stress disorders, PTSD) unterteilt.

In der Regel lassen sich durch die Gabe von Monoaminwiederaufnahrnehemmern gute Behandlungserfolge bei Patienten erzielen, die an Depressionen oder Angststörungen leiden. Dennoch sind ca. 30 % der Patienten refraktär und die Rückfailraten sind hoch, so dass aufgrund dieser eingeschränkten Therapieerfolge und der o. g. häufigen Nebenwirkungen der Monoaminwiederaufnahmehemmer ein dringender Bedarf an einer erfolgreichen und patientengerechten Behandlung von Depressionen und Angststönmgen besteht.

JP 2004 149429 offenbart Indolverbindungen mit β-Sekretase inhibierender Wirkung. Aus WO 99/58490 sind Arylhydronaphthalinalkanamine sowie Arzneimittel enthaltend diese Verbindungen bekannt.

Eine der Erfindung zugrunde liegenden Aufgabe bestand darin, neue Verbindungen zur Verfügung zu stellen, die sich zur Therapie von Schmerzen, Depressionen und/oder Angststörungen eignen. Darüber hinaus sollten diese Verbindungen möglichst wenige Nebenwirkungen der Monoaminwiederaufnahmehemmer wie z.B. Akkomodationsstörungen, Serotoninsyndrom oder QT-Verlängerungen oder der Oplate wie z.B. Aterndepression, Erbrechen, Sedierung, Obstipation und Toleranzentwicklung aufweisen. Weitere Aufgaben bestanden darin, neue Wirkstoffe zur Behandlung von Migräne, Harninkontinenz, Fibromyalgia, Essstörungen, Bulimie, Hyperaktivität, Drogenabhängigkeit, -sucht und -entzug, Trichotillomanie, Tourette's Syndrom, Hauterkrankungen wie Postherpetische Neuralgie und Pruritus, Psychosen, Gedächtnisstörungen, kognitiven Störungen und/oder Morbus Alzheimer zur Verfügung zu stellen.

Es wurde nun gefunden, dass Derivate der nachstehenden allgemeinen Formel I eine hohe Affinität zum μ-

Oplatrezeptor aufweisen und/oder die Wiederaufnahme von Noradrenalin und/oder Serotonin hemmen. Die Substanzen verfügen über ausgeprägte antinociceptive, antidepressive und anxiolytische Wirkungen und sind somit zur Behandlung von Depressionen, Angststörungen und Schmerzen geeignet. Die erfindungsgemäßen Verbindungen besitzen Insbesondere ein Potential zur Therapie von chronischen Schmerzzuständen, die mit depressiven Verstimmungen oder Angststörungen vergesellschaftet sind. Darüber hinaus eignen sich die Substanzen zur Behandlung von Migräne. Haminkontinenz, Fibromyalgia, Essstörungen, Bulimie, Hyperaktivität, Drogenabhängigkeit, -sucht und - entzug, Trichotillomanie, Tourette's Syndrom, Hauterkrankungen wie Postherpetische Neuralgie und Pruritus, Psychosen, Gedächtnisstörungen, kognitiven Störungen und Morbus Alzheimer.

Gegenstand der vorliegenden Erfindung sind daher gesättigte und ungesättigte 3-Pyridyl-benzocycloalkylmethyl-amine der folgenden allgemeinen Formel I, auch in Form ihrer Razemate, Enantiomere, Diastereomere, insbesondere Mischungen ihrer Enantiomere oder Diastereomere oder eines einzelnen Enantiomers oder Diastereomers sowie ihrer freien Basen oder eines mit einer physiologisch vertraglichen Säure gebildeten Salzes:

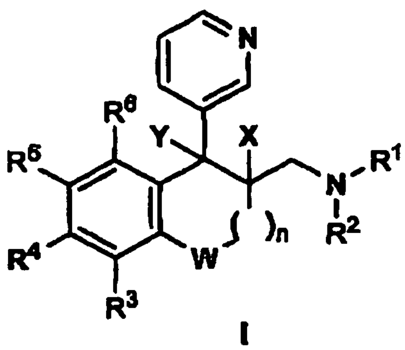

I

, worin

W gleich $CH_2$, O, S, SO oder $SO_2$ ist und n = 0 - 3 ist,

$R^1$ und $R^2$ unabhängig voneinander ausgewählt sind aus H und $C_1$-$C_{10}$-Alkyl, jeweils verzweigt oder unverzweigt, einfach oder mehrfach substituiert oder unsubstituiert;

und

$R^3$ bis $R^8$ unabhängig voneinander aus H oder beliebigen Resten mit Ausnahme von weiteren ankondensierten Ringen ausgewählt sind.

Dabei versteht man unter Resten im Sinne dieser Erfindung die Substitution mindestens eines Wasserstoffrestes durch F, Cl, Br, I, CN, $CF_3$, $OCF_3$, $C_1$-$C_{10}$-Alkyl, jeweils verzweigt oder unverzweigt, einfach oder mehrfach substituiert oder unsubstituiert;

$OR^{6'}$ oder $SR^{6'}$, wobei $R^{6'}$ ausgewählt ist aus

H und $C_1$-$C_{10}$-Alkyl, jeweils verzweigt oder unverzweigt, einfach oder mehrfach substituiert oder unsubstituiert;

In der allgemeinen Formel I ist Y ausgewählt aus H und OH, wenn gleichzeitig X gleich H ist, oder X und Y bilden zusammen eine Bindung.

Unter dem Begriff "subsitituiert" versteht man im Sinne dieser Erfindung die Substitution eines Wasserstoffrestes durch F, Cl, Br, I, $NH_2$, SH oder OH, $OCH_3$, wobei unter mehrfach substituierten Resten Reste zu verstehen sind, die sowohl an verschiedenen als auch an gleichen Atomen mehrfach substituiert sind, beispielsweise dreifach am gleichen C-Atom wie im Falle von $CF_3$ oder an verschiedenen Stellen wie im Falle von $-CH(OH)-CH=CH-CHCl_2$.

Der Ausdruck "$C_1$-$C_{10}$-Alkyl" bedeutet im Sinne dieser Erfindung Kohlenwasserstoffe mit 1 bis 10 Kohlenstooffatomen. Beispielhaft seien Methyl, Ethyl, Propyl, Isopropyl, n-Butan, sek. Butyl, tert. Butyl, n-Pentan, Neopentyl, n-Hexan, n-Heptan, n-Octan, n-Nonan, n-Decan genannt.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind.

Bevorzugt sind Verbindungen der allgemeinen Formel I, in der n < 3 ist. Besonders bevorzugt sind Verbindungen der allgemeinen Formel I, in der W = $CH_2$ und n < 3 ist.

Ganz besonders bevorzugt sind Verbindungen der allgemeinen Format I, in der W = $CH_2$ ist, n < 3 ist, $R^1$ und $R^2$ = Methyl sind und $R^5$ und $R^6$ = H bedeuten.

Wiederum bevorzugt sind folgende erfindungsgemäße Verbindungen sowie deren Salze:

2-Dimethylaminomethyl-1-pyridin-3-yl-indan-1-ol und das entsprechende Hydrochlorid (polares Diastereomer) **(1)**

2-Dimethylaminomethyl-1-pyridin-3-yl-indan-1-ol und das entsprechende Hydrochlorid (unpolares Diastoreomer) **(2)**

Dimethyl-(3-pyridin-3-yl-1*H*-inden-2-ylmethyl)-amin und das entsprechende Hydrochlorid **(3)**

Dimethyl-(1-pyridin-3-yl-indan-2-ylmethyl)-amin und das entsprechende Hydrochlorid **(4)**

2-Dimethylaminomethyl-5-methoxy-1-pyridin-3-indan-1-ol und das entsprechende Hydrochlorid (polares und unpolares Diastereomer) **(5)** (6-Methoxy-3-pyridin-3-yl-1*H*-inden-2-ylmethyl)-dimethyl-amin und das entsprechende Hydrochlorid **(6)**

2-Dimethylaminomethyl-1-pyridin-3-yl-3*H*-inden-5-ol und das entsprechende Hydrochlorid **(7)**

2-Dimethylaminomethyl-1-pyridin-3-yl-1,2,3,4-tetrahydro-naphthalin-1-ol und das entsprechende Hydrochlorid (polares und unpolares Diastereomer) **(8)**

Dimethyl-(1-pyridin-3-yl-3,4-dihydro-naphthalin-2-ylmethyl)-amin und das entsprechende Hydrochlorid **(9)**

Dimethyl-(1-pyridin-3-yl-1,2,3,4-tetrahydro-naphthalin-2-ylmethyl)-amin und das entsprechende Hydrochlorid **(10)**

6-Dimethylaminomethyl-5-pyridin-3-yl-6,7,8,9-tetrahydro-5*H*-benzocyclohepten-5-ol und das entsprechende Hydrochlorid **(11)**

Dimethyl-(5-pyridin-3-yl-8,9-dihydro-7*H*-benzocyclohepten-6-ylmethyl)-amin und das entsprechende Hydrochlorid **(12)**

4-Dimethylaminomethyl-5-pyridin-3-yl-2,3,4,5-tetrahydro-benzo[b]thiepin-5-ol und das entsprechende Hydrochlorid (polares Diastereomer) **(13)**

4-Dimethylaminomethyl-5-pyridin-3-yl-2,3,4,5-tetrahydro-benzo[b]thiepin-5-ol und das entsprechende Hydrochlorid (unpolares Diastereomer) **(14)**

Dimethyl-(5-pyridin-3-yl-2,3-dihydro-benzo[b]thiepin-4-ylmethyl)-amin und das entsprechende Hydrochlorid **(15)**

Dimethyl-(1-oxo-5-pyridin-3-yl-2,3-dihydro-1*H*-1λ4-benzo[*b*]thiepin-4-ylmethyl)-amin und das entsprechende Hydrochlorid **(16)**

4-Dimethylaminomethyl-1,1-dioxo-5-pyridin-3-yl-2,3,4,5-tetrahydro-1*H*-1λ6-benzo[*b*]thiepin-5-ol und das entsprechende Hydrochlorid **(17)**

2-Dimethylaminomethyl-5-fluor-1-pyridin-3-yl-indan-1-ol und das entsprechende Hydrochlorid (polares und unpolares Diastereomer) **(18)**

(6-Fluor-3-pyridin-3-yl-1*H*-inden-2-ylmethyl)-dimethyl-amin und das entsprechende Hydrochlorid **(19)**

2-Dimethylaminomethyl-1-pyridin-3-yl-5-trifluormethoxy-indan-1-ol und das entsprechende Hydrochlorid (polares und unpolares Diastereomer) **(20)**

Dimethyl-(3-pyridin-3-yl-6-trifluormethoxy-1*H*-inden-2-ylmethyl)-amin und das entsprechende Hydrochlorid **(21)**

2-Dimethylaminomethyl-1-pyridin-3-yl-5-trifluormethyl-indan-1-ol und das entsprechende Hydrochlorid (polares und unpolares Diastereomer) **(22)**

Dimethyl-(3-pyridin-3-yl-6-trifluormethyl-1*H*-inden-2-ylmethyl)-amin und das entsprechende Hydrochlorid **(23)**

4-Dimethylaminomethyl-5-pyridin-3-yl-2,3,4,5-tetrahydro-benzo[b]oxepin-5-ol und das entsprechende Hydrochlorid (unpolares Diastereomer) **(24)**

4-Dimethylaminomethyl-5-pyridin-3-yl-2,3,4,5-tetrahydro-benzo[*b*]oxepin-5-ol und das entsprechende Hydrochlorid (polares Diastereomer) **(25)**

Dimethyl-(5-pyridin-3-yl-2,3-dihydro-benzo[b]oxepin-4-ylmethyl)-amin und das entsprechende Hydrochlorid **(26)**

3-Dimethylaminomethyl-4-pyridin-3-yl-chroman-4-ol und das entsprechende Hydrochlorid (unpolares Diastereomer) **(27)**

3-Dimethylaminomethyl-4-pyridin-3-yl-chroman-4-ol und das entsprechende Hydrochlorid (polares Diastereomer) **(28)**

Dimethyl-(4-pyridin-3-yl-2*H*-chromen-3-ylmethyl)-amin und das entsprechende Hydrochlorid **(29)**

3-Dimethylaminomethyl-4-pyridin-3-yl-thiochroman-4-ol und das entsprechende Hydrochlorid (unpolares Diastereomer) **(30)**

3-Dimethylaminomethyl-4-pyridin-3-yl-thiochroman-4-ol und das entsprechende Hydrochlorid (polares Diastereomer) **(31)**

Dimethyl-(4-pyridin-3-yl-2*H*-thiochromen-3-ylmethyl)-amin und das entsprechende Hydrochlorid **(32)**

2-Dimethylaminomethyl-6-methoxy-1-pyridin-3-yl-indan-1-ol und das entsprechende Hydrochlorid (unpolares Diastereomer) **(33)**

2-Dimethylaminomethyl-6-methoxy-1-pyridin-3-yl-indan-1-ol und das entsprechende Hydrochlorid (polares Diastereomer) **(34)**

(5-Methoxy-3-pyridin-3-yl-1*H*-inden-2-ylmethyl)-dimethyl-amin und das entsprechende Hydrochlorid **(35)**

2-Dimethylaminomethyl-5-methoxy-1-pyridin-3-yl-1,2,3,4-tetrahydronaphthalin-1-ol und das entsprechende Hydrochlorid (unpolares Diastereomer) **(36)**

2-Dimethylaminomethyl-5-methoxy-1-pyridin-3-yl-1,2,3,4-tetrahydronaphthalin-1-ol und das entsprechende Hydrochlorid (polares Diastereomer) **(37)**

(5-Methoxy-1-pyridin-3-yl-3,4-dihydro-naphthalin-2-ylmethyl)-dimethyl-amin und das entsprechende Hydrochlorid **(38)**

6-Dimethylaminomethyl-5-pyridin-3-yl-7,8-dihydro-naphthalin-1-ol und das entsprechende Hydrochlorid **(39)**

Aus dieser Aufzählung wiederum besonders bevorzugt sind die Verbindungen:

Dimethyl-(3-pyridin-3-yl-1*H*-inden-2-ylmethyl)-amin und das entsprechende Hydrochlorid **(3)**

(6-Methoxy-3-pyridin-3-yl-1*H*-inden-2-ylmethyl)-dimethyl-amin und das entsprechende Hydrochlorid **(6)**

2-Dimethylaminomethyl-1-pyridin-3-yl-3*H*-inden-5-ol und das entsprechende Hydrochlorid **(7)**

6-Dimethylaminomethyl-5-pyridin-3-yl-6,7,8,9-tetrahydro-5*H*-benzocyclohepten-5-ol und das entsprechende Hydrochlorid **(11)**

Dimethyl-(5-pyridin-3-yl-8,9-dihydro-7*H*-benzocyclohepten-6-ylmethyl)-amin und das entsprechende Hydrochlorid **(12)**

(6-Fluor-3-pyridin-3-yl-1*H*-inden-2-ylmethyl)-dimethyl-amin und das entsprechende Hydrochlorid **(19)**

Dimethyl-(3-pyridin-3-yl-6-trifluormethoxy-1*H*-inden-2-ylmethyl)-amin und das entsprechende Hydrochlorid **(21)**

(5-Methoxy-1-pyridin-3-yl-3,4-dihydro-naphthalin-2-ylmethyl)-dimethyl-amin und das entsprechende Hydrochlorid **(38)**

6-Dimethylaminomethyl-5-pyridin-3-yl-7,8-dihydro-naphthalin-1-ol und das entsprechende Hydrochlorid **(39)**

Ein weiterer Gegenstand der vorliegenden Erfindung ist ein Verfahren zur Herstellung von gesättigten und ungesättigten 3-Pyridyl-benzocycloalkylmethyl-aminen der oben angegebenen allgemeinen Formel **I.**

3-Pyridyl-benzocycloalkylmethyl-amine der allgemeinen Formel **I** werden hergestellt, indem man zunächst Cycloalkanone der allgemeinen Formel **II** mit Immoniumsalzen der Formel **III** bzw. mit Paraformaldehyd und einem Amin der Formel **IV** umsetzt. Anstelle der Immoniumsalze der Formel **III** können auch die für deren Herstellung üblicherweise eingesetzten Reagenzien, z.B. Bis-(dimethylamino)-methan und Acetylchlorid, in an sich bekannter Weise eingesetzt werden (A. Geronikaki et al. Pharmazie 1989, 44, 349). $R^{10}$ hat eine $R^1$ analoge Bedeutung, $R^{11}$ eine $R^2$ analoge Bedeutung. Cycloalkanone der allgemeinen Formel **II** sind entweder kommerziell erhältlich oder lassen sich nach dem Fachmann bekannten Verfahren herstellen.

Anschließend setzt man die so erhaltenen Mannich-Basen mit einer metallorganischen Verbindung der Formel **V** um, in der Z Lithium bedeutet. Die Umsetzung der Mannich-Basen mit einer lithiumorganischen Verbindung der Formel **V,** in der Z Li bedeutet, kann in einem aliphatischen Ether beispielsweise Diethylether und/oder Tetrahydrofuran bei Temperaturen zwischen -70°C und 60°C durchgeführt werden. Im Falle, dass entweder $R^{10}$ oder $R^{11}$ oder beide gleichzeitig gleich Wasserstoff sind, setzt man in die Mannich-Reaktion Verbindungen der allgemeinen Formel **III** bzw. **IV** ein, in denen $R^{10}$ oder $R^{11}$ oder $R^{10}$ und $R^{11}$ einen Benzylrest darstellen. Dieser wird an geeigneter Stelle der Reaktionssequenz durch Umsetzung der entsprechenden Verbindungen mit katalytisch aktiviertem Wasserstoff, wobei Platin oder Palladium, absorbiert auf einem Trägermaterial wie Aktivkohle, als Katalysator dienen, entfernt.

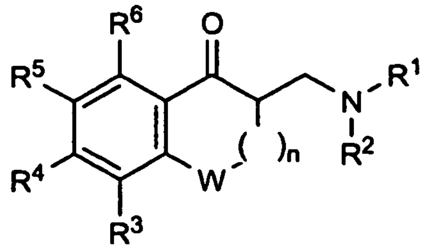

**II**

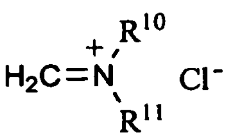

$NHR^{10}R^{11}$

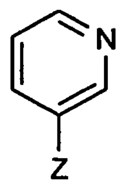

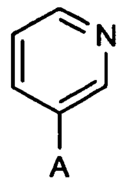

**III** **IV** **V** **VI**

Verbindungen der Formel **V,** in der Z Lithium oder Magnesium-Halogenid bedeutet, lassen sich durch Umsetzung von Halogenverbindungen der Formel **VI,** in der A gleich Cl, Br oder I bedeutet, mit beispielsweise n-Butyllithium/Hexan-Lösung durch Halogen-Lithiumaustausch erhalten. Verbindungen der Formel **V** können auch in Gegenwart von z.B. Cer(III)halogenid mit Verbindungen der Formel **II** umgesetzt werden. Man erhält so zunächst Produkte der allgemeinen Formel **VII,** in der $R^{10}$ eine $R^1$ analoge Bedeutung und $R^{11}$ eine $R^2$ analoge Bedeutung hat. Durch Umsetzung von Verbindungen der allgemeinen Formel **VII** mit Thionylchlorid und anschließender basischer Aufarbeitung erhält man die Verbindungen der Formel **IX.** In manchen Fällen tritt ein Gemisch aus Verbindungen der allgemeinen Formeln **VIII** und **IX** auf, in denen $R^{10}$ eine $R^1$ analoge Bedeutung und $R^{11}$ eine $R^2$ analoge Bedeutung hat. Diese können säulenchromatographisch oder durch Kristallisation getrennt werden. Gezielt können Verbindungen der allgemeinen Formel **IX** durch Umsetzungen von Verbindungen der allgemeinen Formel **VII** mit starken Säuren, z. B. Salzsäure oder Bromwasserstoffsäure erhalten werden. Bevorzugt kann Bromwasserstoffsäure hierfür verwendet werden, wenn dabei gleichzeitig die Demethylierung eines Methoxy-Restes $R^3$ bis $R^6$ angestrebt wird. Alternativ können für Verbindungen der

allgemeinen Formel **IX** in einem nachgeschalteten Reaktionsschritt andere, dem Fachmann geläufige Verfahren für die Demethylierung eines Methoxy-Restes $R^3$ bis $R^6$, wie z.B. Erhitzen in Gegenwart eines Überschusses an Diisobutylaluminiumhydrid in z.B. Toluol oder unter Verwendung von Diphenylphosphin und einer Alkyllithiumverbindung in z.B. Toluol/Tetrahydrofuran, angewendet werden.

Anschließende hydrogenolytische Spaltung von Verbindungen der allgemeinen Formel **VIII** oder Hydrierung von Verbindungen der allgemeinen Formel **IX** , in der $R^{10}$ eine $R^1$ analoge Bedeutung und $R^{11}$ eine $R^2$ analoge Bedeutung hat, mit katalytisch aktiviertem Wasserstoff, wobei Platin oder Palladium, absorbiert auf einem Trägermaterial wie Aktivkohle, als Katalysator dienen, führt zu Verbindungen der Formel **X,** in der $R^{10}$ eine $R^1$ analoge Bedeutung und $R^{11}$ eine $R^2$ analoge Bedeutung hat. Die Hydrierung wird in einem Lösungsmittel wie Essigsäureethylester oder einem $C_1$ - $C_4$-Alkylalkohol bei Drücken von 0,1 bis 10 bar und Temperaturen von 20°C bis 80°C durchgeführt.
Ist W gleich S, so können diese Verbindungen an geeigneter Stelle der Reaktionssequenz mit einem Oxidationsmittel wie $H_2O_2$ in die entsprechenden SO- bzw. $SO_2$ Verbindungen übergeführt werden.

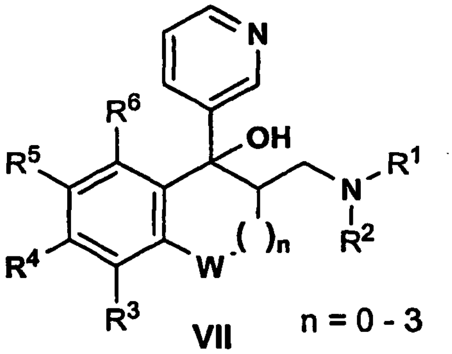

**VII** n = 0 - 3

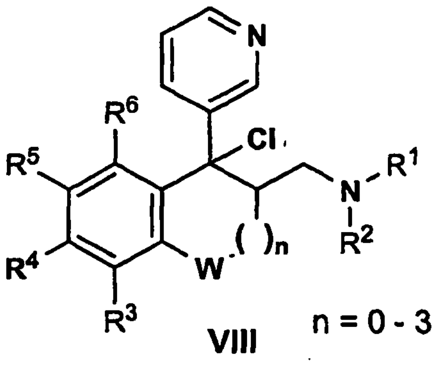

**VIII** n = 0 - 3

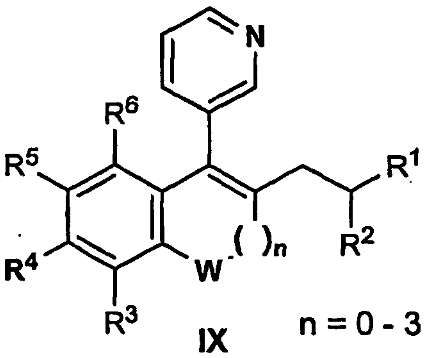

**IX** n = 0 - 3

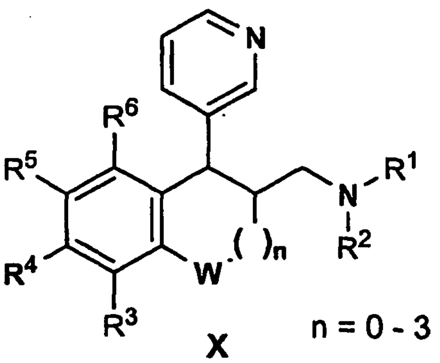

**X** n = 0 - 3

Ein alternatives Verfahren zur Herstellung von ungesättigten 3-Pyridyl-benzocycloalkylmethyl-aminen der oben angegebenen allgemeinen Formel **I**, in denen X und Y zusammen eine Bindung bilden, ist die Übergangsmetall-katalysierte Kreuzkopplung von Verbindungen der allgemeinen Formel **XI,** in der $G_2$ gleich Cl, Br, I, $Sn(Alkyl)_3$ oder $OSO_2CF_3$ bedeutet, mit Verbindungen der allgemeinen Formel **XII,** in der $G_1$ gleich Cl, Br, I oder $B(OR^x)_2$ bedeutet, mit $R^x$ ausgewählt aus H oder Alkyl, wobei die Reaktion mit oder ohne Base und mit oder ohne Ligand durchgeführt werden kann. Ein Beispiel hierfür ist die Suzuki-Kopplung von Verbindungen der allgemeinen Formel **XI,** in denen $G_2$ $OSO_2CF_3$ bedeutet, mit Verbindungen der allgemeinen Formel **XII,** in denen G1 $B(OAlkyl)_2$ bedeutet, in Gegenwart von Palladium(II)acetat, einem Phosphinliganden sowie einer Base.

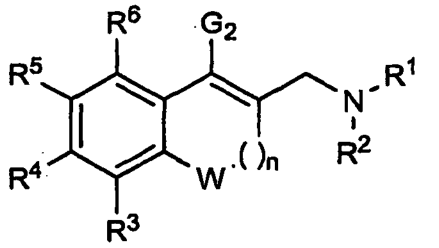
XI

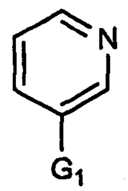
XII

Ein weiteres alternatives Verfahren zur Herstellung der von ungesättigten 3-Pyridyl-benzocycloalkylmethylaminen der oben angegebenen allgemeinen Formel **I**, in denen X und Y zusammen eine Bindung bilden und in denen W gleich O oder S sowie n = 1 ist, ist die Behandlung von orthohalogensubstituierten Phenolen/Thiophenolen (mit Hal ausgewählt aus Br, I, $OSO_2CF_3$) der allgemeinen Formel **XIII** mit substituierten Propargylaminen der allgemeinen Formel **XIV** oder mit Propargylalkoholen oder deren Derivaten der allgemeinen Formel **XV** (mit R° ausgewählt aus H oder einer Schutzgruppe), z.B. in Gegenwart von Dichlorbis(triphenylphosphin)-palladium (II), Kupfer(I)iodid sowie Triethylamin. Im Falle der Propargylalkohole oder deren Derivaten der allgemeinen Formel **XV** wird die Funktion OR° in einem nachgeschalteten Reaktionschritt nach dem Fachmann bekannten Verfahren in die Aminofunktion $NR^1R^2$ übergeführt.

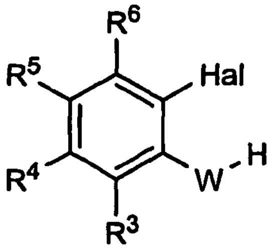
XIII

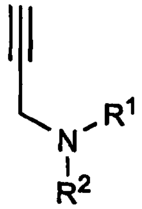
XIV

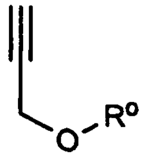
XV

Unter den genannten Reaktionsbedingungen können OH- SH und $NH_2$-Gruppen unerwünschte Nebenreaktionen eingehen. Es ist daher bevorzugt, diese mit Schutzgruppen zu versehen oder im Falle von $NH_2$ durch $NO_2$ zu ersetzen und im letzten Reaktionsschritt die Schutzgruppe abzuspalten, bzw. die $NO_2$-Gruppe zu reduzieren. Ein weiterer Gegenstand der Erfindung ist daher eine Abwandlung des oben beschriebenen Verfahrens, bei dem mindestens eine in Formel I enthaltene OH-Gruppe durch eine $OSi(Ph)_2$tert.but.-Gruppe, mindestens eine SH-Gruppe durch eine S-p-Methoxybenzylgruppe und/oder mindestens eine $NH_2$-Gruppe durch eine $NO_2$-Gruppe ersetzt wurde und nach Abschluss der gesamten Reaktionssequenz eine $OSi(Ph)_2$tert.butyl-Gruppe mit Tetrabutylammoniumfluorid in Tetrahydrofuran und/oder mindestens eine p-Methoxybenzylgruppe mit einem Metallamin, bevorzugt Natriumamin, abgespalten und/oder mindestens eine $NO_2$-Gruppe zu $NH_2$ reduziert wird.

Weiter sind Carbonsäure- oder Thiocarbonsäure-Gruppen unter den Bedingungen der Butyllithium-Reaktion unter Umständen nicht stabil, so dass es bevorzugt ist, deren Methylester umzusetzen und das Verfahrensprodukt aus der Butyllithium-Reaktion im letzten Reaktionsschritt mit KOH-Lösung bzw. NaOH-Lösung in Methanol bei 40°C - 60°C zu verseifen. Ein weiterer Gegenstand der Erfindung ist daher eine Abwandlung der oben beschriebenen Verfahren, in dem nach der Butyllithium-Reaktion ein Verfahrensprodukt mit mindestens einer $C(O)OCH_3$- und/oder $C(S)OCH_3$-Gruppe mit KOH-Lösung bzw. NaOH-Lösung in Methanol bei 40°C - 60°C verseift wird.

Die Reinigung der in den einzelnen Reaktionssequenzen erhaltenen Verbindungen erfolgt durch Kristallisation oder Säulenchromatographie.

Die Verbindungen der Formel I lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Maleinsäure, Glutaminsäure und/oder Asparaginsäure in an sich bekannter Weise in ihre Salze überführen. Vorzugsweise wird die Salzbildung in einem Lösungsmittel wie Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

Die erfindungsgemäßen gesättigten und ungesättigten 3-Pyridyl-benzocycloalkylmethyl-amine der allgemeinen Formel I sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße Verbindung der allgemeinen Formel **I** sowie gegebenenfalls physiologisch verträgliche Hilfsstoffe enthalten. Vorzugsweise eignen sich die erfindungsgemäßen Arzneimittel zur Bekämpfung von Schmerzen (insbesondere chronischer Schmerz, neuropathischer Schmerz, Entzündungsschmerz), Migräne, Fibromyalgia oder zur Behandlung von Depressionen (Unipolar, schwere Depression mit und ohne Wahn, mittelgradige Depressionen, leichte Depressionen, Melancholie, bipolare Depressionen; Bipolare Erkrankungen I (Manie und schwere Depressionen), Bipolare Erkrankungen II (Hypomanie und schwere Depressionen) Zyklothyme Persönlichkeitsstörungen (Hypomanie und milde Depressionen), Subtypen), Angstzuständen (Subtypen Generalisierte Angststörungen, Panikattacken, Zwangssyndromen , social anxiety disorder, Phobien, PSTD), Schlafstörungen, Harninkontinenz (Stress- und Drang-), Essstörungen, Bulemie, Attention deficit hyperactivity disorder, Sucht und Abhängigkeit, Trichotillomanie, Tourette's Syndrom, Hauterkrankungen wie Postherpetische Neuralgie und Pruritus ,Psychosen, Gedächtnisstörungen, kognitiven Störungen und/oder Morbus Alzheimer.

Die Verwendung wenigstens einer Verbindung der allgemeinen Formel **I** zur Herstellung eines Arzneimittels zur Bekämpfung von Schmerzen (insbesondere chronischer Schmerz, neuropathischer Schmerz, Entzündungsschmerz), Migräne, Fibromyalgia oder zur Behandlung von Depressionen (Unipolar, schwere Depression mit und ohne Wahn, mittelgradige Depressionen, leichte Depressionen, Melancholie, bipolare Depressionen; Bipolare Erkrankungen I (Manie und schwere Depressionen), Bipolare Erkrankungen II (Hypomanie und schwere Depressionen) Zyklothyme Persönlichkeitsstörungen (Hypomanie und milde Depressionen), Subtypen), Angstzuständen (Subtypen Generalisierte Angststörungen, Panikattacken, Zwangssyndromen, social anxiety disorder, Phobien, PSTD), Schlafstörungen, Harninkontinenz (Stress- und Drang-), Essstörungen, Bulemie, Attention deficit hyperactivity disorder, Sucht und Abhängigkeit, Trichotillomanie, Tourette's Syndrom, Hauterkrankungen wie Postherpetische Neuralgie und Pruritus, Psychosen, Gedächtnisstörungen, kognitiven Störungen und/oder Morbus Alzheimer, ist ebenfalls Gegenstand der vorliegenden Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und als solche auch verabreicht werden.

Neben wenigstens einer erfindungsgemäßen Verbindung der allgemeinen Formel I enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verwindungen der allgemeinen Formel I auch verzögert freisetzen.

Die Herstellung der erfindungsgemäßen Arzneimittel kann mit Hilfe von üblichen, dem Fachmann bekannten Mitteln, Vorrichtungen, Methoden und Verfahren erfolgen, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen gesättigten oder ungesättigten 3-Pyridyl-benzocycloalkylmethyl-amine der allgemeinen Formel I kann variieren und ist beispielsweise abhängig vom Gewicht oder dem Alter des Patienten sowie von der Applikationsart, der Indikation und dem Schweregrad der Erkrankung. Üblicherweise werden 0,005 bis 500 mg/kg, vorzugsweise 0,05 bis 5 mg/kg Körpergewicht des Patienten wenigstens einer erfindungsgemäßen Verbindung der allgemeinen Formel I appliziert.

## Beispiele

Die folgenden Beispiele zeigen erfindungsgemäße Verbindungen sowie deren Darstellung und mit diesen

durchgeführte Wirksamkeitsuntersuchungen.

Dabei gelten generell folgende Angaben:

Die Ausbeuten der hergestellten Verbindungen sind nicht optimiert.

Alle Temperaturen sind unkorrigiert.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Die Angabe Ether bedeutet Diethylether.

***Beispiel 1:***

2-Dimethylaminomethyl-1-pyridin-3-yl-indan-1-ol Hydrochlorid

Zu einer Lösung von 8,8 ml 3-Brompyridin in 130 ml Diethylether p.a. wurden bei einer Temperatur von -35 bis -40 °C 37 ml n-Butyllithiumlösung (2,5 mol/l in Hexan) zugetropft. Nach weiteren 60 Minuten bei dieser Temperatur wurden bei fortgesetzter Kühlung 11,1 g Indan-1-on, gelöst in ca. 20 ml Diethylether p.a. zugetropft und über Nacht bis auf Raumtemperatur erwärmt. Anschließend wurden bei 0 bis 10 °C ca. 30 ml Wasser zugegeben, die Phasen getrennt, die wässrige Phase zweimal mit Tetrahydrofuran/Diisopropylether extrahiert, die vereinigten organischen Phasen über Magnesiumsulfat getrocknet, filtriert und eingeengt. Das erhaltene Rohprodukt (19,9 g) wurde mit Methanol/Essigsäureethylester (V/V = 5:1) an Kieselgel chromatographiert. Es wurden 7,0 g vorgereinigtes Produkt erhalten, die mit Methanol/Essigsäureethylester (V/V = 0:1 bis 5:1) nochmals an Kieselgel chromatographiert wurden. Es wurden 3,54 g des unpolareren Isomers von 2-Dimethylaminomethyl-1-pyridin-3-yl-indan-1-of erhalten, die in ca. 140 ml Aceton gelöst und mit ca. 0,5 ml Wasser und ca. 3,7 ml Chlortrimethylsilan in das korrespondierende Hydrochlorid übergeführt wurden, das mit ca. 30 ml absolutem Ethanol gewaschen und im Vakuum (ca. 50 mbar) getrocknet wurde (Ausbeute 2,64 g, Schmelzpunkt von 195 °C bis 196 °C (Zersetzung)). Neben 1,71 g Mischfraktion wurden 0,86 g des polareren Isomers von 2-Dimethylaminomethyl-1 pyridin-3-yl-indan-1 ol erhalten. Letztere wurden in ca. 90 ml Aceton gelöst und mit ca. 0,13 ml Wasser und ca. 0,90 ml Chlortrimethylsilan in das korrespondierende Hydrochlorid übergeführt (Ausbeute 0,76 g, Schmelzpunkt von 194 °C bis 195 °C (Zersetzung)).

Hiermit wurden ***Beispiele 1 und*** **2** 2-Dimethylaminomethyl-1-pyridin-3-yl-indan-1-ol Hydrochlorid beschrieben.

Analog wurden die Verbindungen der Beispiele 8, 13 und 14 erhalten.

***Beispiel 3:***

Dimethyl-(3-pyridin-3-yl-1*H*-inden-2-ylmethyl)-amin Hydrochlorid

Zu 2,17 g 2-Dimethylaminomethyl-1-pyridin-3-yl-indan-1-ol (Gemisch der Diastereomeren) wurden unter starker Gasentwicklung 2,6 ml Thionylchlorid zugetropft, die Mischung unter Rühren für 2,5 Stunden auf 65 °C erhitzt und anschließend an der Wasserstrahlpumpe eingeengt. Nach Abkühlung wurden zunächst Wasser, dann 1 M Natriumcarbonatlösung zugegeben und anschließend mit Tetrahydrofuran/Ethylacetat extrahiert, die vereinigten Extrakte über Magnesiumsulfat getrocknet, filtriert und eingeengt. Das erhaltene Rohprodukt (1,4 g) wurde in ca. 17 ml absolutem Ethanol gelöst und mit Wasser und Chlortrimethylsilan in das korrespondierende Hydrochlorid übergeführt. Der ausgefallene Feststoff wurde abgesaugt, mit wenig absolutem Ethanol gewaschen und bei 120 °C im Vakuum (ca. 50 mbar) getrocknet. Es wurden 1,74 g Dimethyl-(3-pyridin-3-yl-1*H*-inden-2-ylmethyl)-amin Hydrochlorid mit einem Schmelzpunkt von 156 °C bis 159 °C erhalten.

Hiermit wurde ***Beispiel 3*** Dimethyl-(3-pyridin-3-yl-1*H*-inden-2-ylmethyl)-amin Hydrochlorid beschrieben.

Analog wurde die Verbindung des Beispiels 9 erhalten.

***Beispiel 4:***

Dimethyl-(1-pyridin-3-yl-indan-2-ylmethyl)-amin Hydrochlorid

1,25 g Dimethyl-(3-pyridin-3-yl-1*H*-inden-2-ylmethyl)-amin Hydrochlorid wurden in ca. 13 ml Methanol gelöst,

unter Stickstoff mit 0,63 g 10%igem Palladium auf Tierkohle versetzt und unter 2 bar Wasserstoffatmosphäre 8 h bei Raumtemperatur gerührt. Anschließend wurde unter Stickstoff abgesaugt und eingeengt. Der Rückstand wurde in ca. 12 ml absolutem Ethanol kristallisiert. Das Filtrat wurde mit 1M Natriumcarbonatlösung und Dichlormethan in die freie Base übergeführt und mit Methanol/Dichlormethan (V/V = 1:2) an Kieselgel chromatographiert, wobei eine Mischfraktion sowie eine reine Fraktion erhalten wurden. Die Mischfraktion wurde nochmals mit Methanol/Dichlormethan (V/V = 1:2) an Kieselgel chromatographiert. Insgesamt wurden 0,52 g 2- Dimethyl-(1-pyridin-3-yl-indan-2-ylmethyl)-amin erhalten, die in ca. 5 ml Aceton gelöst und mit Wasser und Chlortrimethylsilan in das korrespondierende Hydrochlorid übergeführt wurden (Ausbeute 0,36 g, Schmelzpunkt von 189 °C bis 191 °C).

Hiermit wurde ***Beispiel 4*** Dimethyl-(1-pyridin-3-yl-indan-2-ylmethyl)-amin Hydrochlorid beschrieben.

Analog wurde die Verbindung von Beispiel 10 erhalten.

***Beispiel 5:***

2-Dimethylaminomethyl-5-methoxy-1-pyridin-3-yl-indan-1-ol Hydrochlorid

Zu einer Lösung von 7,3 ml 3-Brompyridin in ca. 125 ml Diethylether p.a. wurden bei einer Temperatur von -30 bis -35 °C 47 ml n-Butyllithiumlösung (1,6 mol/l in n-Hexan) zugetropft. Nach weiteren 20 Minuten bei dieser Temperatur wurden bei fortgesetzter Kühlung 11 g 2-Dimethylaminomethyl-5-methoxy-indan-1-on, gelöst in Diethylether p.a. zugetropft, 40 min bei dieser Temperatur weitergerührt und über Nacht bis auf Raumtemperatur erwärmt. Anschließend wurden bei -10 bis 0 °C 46 ml Wasser zugegeben, die Phasen getrennt, die wässrige Phase mit Diethylether extrahiert (dünnschichtchromatographische Kontrolle), die vereinigten organischen Phasen mit gesättigter Natriumchloridlösung gewaschen und über Magnesiumsulfat getrocknet, filtriert und eingeengt. Das erhaltene Rohprodukt wurde mit Essigsäureethylester/25%iger Ammoniaklösung (V/V = 98:2) an Kieselgel chromatographiert. Es wurden 7,06 g 2-Dimethylaminomethyl-5-methoxy-1-pyridin-3-yl-indan-1-ol erhalten, die in ca. 125 ml 2-Butanon p.a. gelöst und unter Eiskühlung mit ca. 0,3 ml Wasser und ca.4,2 ml Chlortrimethylsilan in das korrespondierende Hydrochlorid übergeführt wurden, das unter Luftausschluss abgesaugt und im Vakuum (ca. 50 mbar) getrocknet wurde (Ausbeute 6,79 g, Gemisch der beiden Diastereomeren).

Hiermit wurde ***Beispiel 5*** 2-Dimethylaminomethyl-5-methoxy-1-pyridin-3-yl-indan-1-ol Hydrochlorid beschrieben.

Analog wurden die Verbindungen der Beispiele 27, 28, 30, 31, 33 und 34 erhalten.

***Beispiel 6*:**

(6-Methoxy-3-pyridin-3-yl-1*H*-inden-2-ylmethyl-dimethyl-amin Hydrochlorid

Zu 0,837 g 2-Dimethylaminomethyl-5-methoxy-1-pyridin-3-yl-indan-1-ol Hydrochlorid (Gemisch der Diastereomeren) wurden bei Raumtemperatur ca. 9 ml 37%ige Salzsäure zugegeben, über Nacht gerührt und anschließend bei 70 °C im Vakuum eingeengt. Der Rückstand wurde zweimal in Dichlormethan aufgenommen und wieder eingeengt. Der ölige braune Rückstand wurde in 40 ml Aceton aufgenommen und gerührt, worauf sich ein kristalliner Feststoff bildete. Nach weiterem Rühren über 2,5 Tage wurde unter Luftausschluss abgesaugt, zweimal mit Diethylether gewaschen und im Ölpumpenvakuum getrocknet. Es wurden 0,603 g (6Methoxy-3-pyridin-3-yl-1H-inden-2-ylmethyl)-dimethyl-amin Hydrochlorid erhalten.

Hiermit wurde ***Beispiel 6*** 6-Methoxy-3-pyridin-3-yl-1*H*-inden-2-ylmethyl)-dimethyl-amin Hydrochlorid beschrieben.

Analog wurde die Verbindungen der Beispiele 15, 19, 23, 26, 29, 32 und 35 erhalten.

***Beispiel 7:***

2-Dimethylaminomethyl-1-pyridin-3-yl-3*H*-inden-5-ol Hydrochlorid

0,84 g 2-Dimethylaminomethyl-5-methoxy-1-pyridin-3-yl-indan-1-ol Hydrochlorid (Gemisch der Diastereomeren) wurden in ca. 13 ml 33%iger Bromwasserstoffsäure in Essigsäure gelöst und bei 100-110 °C 3 h unter Rückfluss gerührt. Der Reaktionsansatz wurde im Vakuum eingeengt, mit 32%iger Natronlauge alkalisch gemacht, mit Essigsäureethylester extrahiert, die vereinigten organischen Phasen über Magnesiumsulfat getrocknet, mit Aktivkohle gerührt, filtriert und eingeengt. Es wurden 0,62 g 2-Dimethylaminomethyl-1-pyridin-3-yl-3*H*-inden-5-ol erhalten, die in ca.22 ml 2-Butanon/Aceton (V/V = 1:2) gelöst und unter Eiskühlung mit ca. 0,04 ml Wasser und ca. 0,58 ml Chlortrimethylsilan in das korrespondierende Hydrochlorid übergeführt wurden, das unter Luftausschluss abgesaugt, mit Diisopropylether gewaschen und im Vakuum (ca. 50 mbar) getrocknet wurde (Ausbeute 0,631 g)

Hiermit wurde ***Beispiel 7*** 2-Dimethylaminomethyl-1-pyridin-3-yl-3*H*-inden-5-ol Hydrochlorid beschrieben.

***Beispiel 11:***

6-Dimethylaminomethyl-5-pyridin-3-yl-6,7,8,9,tetrahydro-5*H*-benzocyclohepten-5-ol Hydrochlorid

Zu einer Lösung von 12,0 ml 3-Brompyridin in ca. 180 ml Diethylether p.a. wurden bei einer Temperatur von -35 bis -40 °C 50 ml n-Butyllithiumlösung (2,5 mol/l in Hexan) zugetropft. Nach weiteren 60 Minuten bei dieser Temperatur wurden bei fortgesetzter Kühlung 14,2 g 6-Dimethylaminomethyl-6,7,8,9-tetrahydro-benzocyclohepten-5-on, gelöst in ca. 70 ml Diethylether p.a. zugetropft und über Nacht bis auf Raumtemperatur erwärmt. Anschließend wurden bei 0 bis 10 °C ca. 30 ml Wasser zugegeben, die Phasen getrennt, die wässrige Phase zweimal mit Tetrahydrofuran/ Diisopropylether extrahiert, die vereinigten organischen Phasen über Magnesiumsulfat getrocknet, filtriert und eingeengt. Das erhaltene Rohprodukt (25,9 g) wurde mit Methanol/Essigsäureethylester (V/V = 1:1) an Kieselgel chromatographiert. Es wurden 17,9 g Produkt erhalten, die in absolutem Ethanol gelöst und mit ca. 2,2 ml Wasser und ca. 15,5 ml Chlortrimethylsilan in das korrespondierende Hydrochlorid übergeführt wurden, das im Vakuum (ca. 50 mbar) getrocknet wurde (Ausbeute 6,6 g, Schmelzpunkt 260 °C (Zersetzung)).

Hiermit wurde ***Beispiel 11*** 6-Dimethylaminomethyl-5-pyridin-3-yl-6,7,8,9-tetrahydro-5*H*-benzocyclohepten-5-ol Hydrochlorid beschrieben.

***Beispiel 12:***

Dimethyl-(5-pyridin-3-yl-8,9-dihydro-7*H*-benzocyclohepten-6-ylmethyl)-amin Hydrochlorid

Zu 5,5 g 6-Dimethylaminomethyl-5-pyridin-3-yl-6,7,8,9-tetrahydro-5H-benzocyclohepten-5-ol Hydrochlorid wurden unter starker Gasentwicklung 12 ml Thionylchlorid zugetropft, die Mischung unter Rühren für 2 Stunden auf 50 °C erhitzt und anschließend an der Wasserstrahlpumpe eingeengt. Nach Abkühlung wurden zunächst 2 N Natronlauge zugegeben und anschließend mit Tetrahydrofuran/Essigsäureethylester extrahiert, die vereinigten Extrakte über Magnesiumsulfat getrocknet, filtriert und eingeengt. Das erhaltene Rohprodukt (2,1 g) wurde mit Methanol/Essigsäureethylester (V/V = 1:1) an Kieselgel chromatographiert. Es wurden 2,01 g vorgereinigtes Produkt erhalten, die mit Methanol/ Essigsäureethylester/n-Hexan (V/V/V = 1:1:1) nochmals an Kieselgel chromatographiert wurden. Es wurden 1,25 g erhalten, die in ca. 10 ml Ethanol absolut/Diisopropylether (V/V = 1:1) gelöst und mit ca. 0,16 ml Wasser und ca. 1,15 ml Chlortrimethylsilan in das korrespondierende Hydrochlorid übergeführt wurden. Der ausgefallene Feststoff wurde abgesaugt, mit wenig absolutem Ethanol gewaschen und im Vakuum (ca. 50 mbar) getrocknet. Es wurden 0,449 g Dimethyl-(5-pyridin-3-yl-8,9-dihydro-7*H*-benzocyclohepten-6-ylmethyl)-amin Hydrochlorid mit einem Schmelzpunkt von 259°C bis 260 °C erhalten.

Hiermit wurde ***Beispiel 12*** Dimethyl-(5-pyridin-3-yl-8,9-dihydro-7*H*-benzocyclohepten-6-ylmethyl)-amin Hydrochlorid beschrieben.

***Beispiel 16:***

Dimethyl-(1-oxo-5-pyridin-3-yl-2,3-dihydro-1*H*-1λ4-benzo[b]thiepin-4-ylmethyl)-amin Hydrochlorid

0,5 g Dimethyl-(5-pyridin-3-yl-2,3-dihydro-benzo[*b*]thiepin-4-ylmethyl)-amin Hydrochlorid wurden in 2,5 ml Eisessig gelöst, unter Rühren bei Raumtemperatur 0,29 ml 35%ige Wasserstoffperoxid-Lösung zugetropft und 24 h weitergerührt. Der Reaktionsansatz wurde unter Eiskühlung mit Essigsäureethylester überschichtet, mit 32%iger Natronlauge alkalisch gemacht, dreimal mit Essigsäureethylester extrahiert, die vereinigten organischen Phasen über Magnesiumsulfat getrocknet, filtriert und im Vakuum eingeengt. Es wurden 0,35 g Rohprodukt erhalten, die mittels präparativer HPLC (Hypercarb 5μm 120 x 4 mm, 0,5 ml/min, Acetonitril/Wasser (V/V = 70:30 + 0,1% Diethylamin)) aufgereinigt wurden. Nach Filtration durch eine MF-Millipore-MCE-Membran (gemischte Celluloseester; 0,45 μm) wurden 0,102 g Dimethyl-(1-oxo-5-pyridin-3-yl-2,3-dihydro-1*H*-1λ4-benzo[*b*]thiepin-4-ylmethyl)-amin erhalten, die in 3 ml 2-Aceton p.a. gelöst und unter Eiskühlung mit ca. 0,003 ml Wasser und ca. 0,041 ml Chlortrimethylsilan in das korrespondierende Hydrochlorid übergeführt wurden, das im Vakuum (ca. 50 mbar) getrocknet wurde (Ausbeute 0,1 g,).

Hiermit wurde ***Beispiel 16*** Dimethyl-(1-oxo-5-pyridin-3-yl-2,3-dihydro-1*H*-1λ4-benzo[*b*]thiepin-4-ylmethyl)-amin Hydrochlorid beschrieben.

***Beispiel 17:***

4-Dimethylaminomethyl-1,1-dioxo-5-pyridin-3-yl-2,3,4,5-tetrahydro-1*H*-1λ6-benzo[*b*]thiepin-5-ol Hydrochlorid

0,5 g 4-Dimethylaminomethyl-5-pyridin-3-yl-2,3,4,5-tetrahydro-benzo[*b*]thiepin-5-ol Hydrochlorid wurden in 2,5 ml Eisessig gelöst, unter Rühren bei Raumtemperatur ca. 0,23 ml 35%ige Wasserstoffperoxid-Lösung zugetropft und 24 h weitergerührt. Der Reaktionsansatz wurde unter Eiskühlung mit Essigsäureethylester überschichtet, mit 32%iger Natronlauge alkalisch gemacht, zweimal mit Essigsäureethylester extrahiert, die vereinigten organischen Phasen mit gesättigter Natriumchloridlösung gewaschen, über Magnesiumsulfat getrocknet, filtriert und im Vakuum eingeengt. Es wurden 0,38 g Rohprodukt erhalten, die mit Essigsäureethylester/25%iger Ammoniaklösung (V/V = 98:2) an Kieselgel chromatographiert wurden. Es wurden 0,15 g einer Mischfraktion sowie 0,13 g Dimethyl-(1-oxo-5-pyridin-3-yl-2,3-dihydro-1*H*-1λ4-benzo[*b*]thiepin-4-ylmethyl)-amin erhalten. Letztere wurden in 6 ml 2-Aceton p.a. gelöst und unter Eiskühlung mit ca. 0,004 ml Wasser und ca. 0,043 ml Chlortrimethylsilan in das korrespondierende Hydrochlorid übergeführt, das im Vakuum (ca. 50 mbar) getrocknet wurde (Ausbeute 0,09 g weißer Feststoff).

Hiermit wurde ***Beispiel 17*** 4-Dimethylaminomethyl-1,1-dioxo-5-pyridin-3-yl-2,3,4,5-tetrahydro-1*H*-1λ6-benzo [*b*]thiepin-5-ol Hydrochlorid beschrieben.

***Beispiel 18:***

2-Dimethylaminomethyl-5-fluor-1-pyridin-3-yl-indan-1-ol; Hydrochlorid

Stufe 1:

2-Dimethylaminomethyl-6-fluor-Indan-1-on Hydrochlorid

2 g 5-Fluor-indan-1-on wurden in 10,5 ml Acetonitril gelöst, 1,84 ml Bisdimethylaminomethan zugegeben und 0,95 ml Acetylchlorid bei 0 bis 10 °C zugetropft. Es wurde 3 h bei 50 °C und anschließend 18 h bei Raumtemperatur gerührt. Der entstandene Niederschlag wurde abfiltriert, einmal mit Acetonitril/Isopropylether (V/V = 1:1) und zweimal mit Aceton gewaschen und im Vakuum getrocknet (Ausbeute 2,9 g).

Stufe 2:

2-Dimethylaminomethyl-5-fluor-indan-1-on

2,8 g 2-Dimethylaminomethyl-5-fluor-indan-1-on Hydrochlorid wurden in Wasser gelöst, unter Eiskühlung mit 32%iger Natronlauge auf pH 12 gestellt, dreimal mit Dichlormethan extrahiert und die vereinigten organischen Phasen über Magnesiumsulfat getrocknet, filtriert und im Vakuum eingeengt.

Stufe 3:

2-Dimethylaminomethyl-5-fluor-1-pyridin-3-yl-indan-1-ol; Hydrochlorid

Zu einer Lösung von 1,55 ml 3-Brompyridin in ca. 28 ml Diethylether p.a. wurden bei einer Temperatur von -35 bis -40 °C ca. 10 ml n-Butyllithiumlösung (1,6 mol/l in n-Hexan) zugetropft. Nach weiteren 20 Minuten bei dieser Temperatur wurden bei fortgesetzter Kühlung 2,2 g des Produkts aus Stufe 2, gelöst in Diethylether p.a. zugetropft, 40 min bei dieser Temperatur weitergerührt und über Nacht bis auf Raumtemperatur erwärmt. Anschließend wurden bei -10 bis 0 °C 10,5 ml Wasser zugegeben, die Phasen getrennt, die wässrige Phase dreimal mit Diethylether extrahiert (dünnschichtchromatographische Kontrolle), die vereinigten organischen Phasen mit gesättigter Natriumchloridlösung gewaschen und über Magnesiumsulfat getrocknet, filtriert und eingeengt. Das erhaltene Rohprodukt wurde mit Essigsäureethylester/25%iger Ammoniaklösung (V/V = 98:2) an Kieselgel chromatographiert. Es wurden 1,5 g 2-Dimethylaminomethyl-5-fluor-1-pyridin-3-yl-indan-1-ol erhalten, die in ca. 50 ml Aceton p.a. gelöst und unter Eiskühlung mit ca. 0,047 ml Wasser und ca. 0,665 ml Chlortrimethylsilan in das korrespondierende Hydrochlorid übergeführt wurden, das unter Luftausschluss abgesaugt und im Vakuum (ca. 50 mbar) getrocknet wurde (Ausbeute 1,05 g, Gemisch der beiden Diastereomeren).

Hiermit wurde ***Beispiel 18*** 2-Dimethylaminomethyl-5-fluor-1-pyridin-3-yl-indan-1-ol Hydrochlorid beschrieben.

***Beispiel 19:***

(6-Fluor-3-pyridin-3-yl-1*H*-inden-2-ylmethyl)-dimethyl-amin Hydrochlorid

Zu 0,5 g 2-Dimethylaminomethyl-5-fluor-1-pyridin-3-yl-indan-1-ol Hydrochlorid (Gemisch der Diastereomeren) wurden bei Raumtemperatur ca. 5,4 ml 37%ige Salzsäure zugegeben, über Nacht gerührt und anschließend im Vakuum eingeengt. Der Rückstand wurde zweimal in Dichlormethan aufgenommen und wieder eingeengt. Der Rückstand wurde in 15 ml Aceton aufgenommen und 2 h gerührt, worauf sich ein kristalliner Feststoff bildete, der unter Luftausschluss abgesaugt, zweimal mit Diethylether gewaschen und im Ölpumpenvakuum bei 60 °C getrocknet wurde. Es wurden 0,39 g (6-Fluor-3-pyridin-3-yl-1*H*-inden-2-ylmethyl)-dimethyl-amin Hydrochlorid erhalten.
Hiermit wurde ***Beispiel 19*** (6-Fluor-3-pyridin-3-yl-1*H*-inden-2-ylmethyl)-dimethyl-amin Hydrochlorid beschrieben.

***Beispiel 20:***

2-Dimethylaminomethyl-1-pyridin-3-yl-5-trifluormethoxy-indan-1-ol Hydrochlorid

5-Trifluormethoxy-indan-1-on wurde in Abweichung von der Literatur (US6159996 A1, 12.12.2000) wie folgt hergestellt:

Stufe 1:

3-(3-Trifluormethoxy-phenyl)-propionsäure

Zu 15 g 3-(Trifluormethoxy)-zimtsäure in ca. 125 ml Essigsäureethylester p.a. wurden unter Stickstoffatmosphäre 0,69 g 5% Palladium auf Aktivkohle gegeben und anschließend 18 h bei Raumtemperatur bei 2 bar Wasserstoffdruck hydriert. Anschließend wurde über Kieselgur filtriert, dreimal mit Essigsäureethylester gewaschen und die vereinigten Filtrate bei 25 bis 40 °C im Vakuum eingeengt (Ausbeute 15,8 g).

Stufe 2:

5-Trifluormethoxy-indan-1-on

12,4 g des Produkts aus Stufe 1 wurden bei ca. 5 °C unter Rühren mit ca. 37 ml Trifluormethansulfonsäure versetzt und anschließend 18 h bei Raumtemperatur gerührt. Anschließend wurde zerstoßenes Eis hinzugegeben und mit Diethylether extrahiert. Die vereinigten organischen Phasen wurden mit gesättigter Natriumhydrogencarbonatlösung, Wasser und gesättigter Natriumchloridlösung gewaschen und über Magnesiumsulfat getrocknet, filtriert und im Vakuum bei 25 bis 40 °C eingeengt (Ausbeute 10,1 g).

Stufe 3:

2-Dimethylaminomethyl-5-trifluormethoxy-indan-1-on Hydrochlorid

Zu einer Lösung von 10,1 g des Produkts aus Stufe 2 in 37 ml Acetonitril wurden 6,4 ml Bis-(dimethylamino)-methan gegeben, bei 0 bis 10°C 3,3 ml Essigsäurechlorid zugetropft, weitere 25 ml Acetonitril hinzugefügt, 3 h bei 50 °C und anschließend 18 h bei Raumtemperatur gerührt. Der entstandene Feststoff wurde abgesaugt, zweimal mit Diethylether gewaschen und im Vakuum getrocknet (Ausbeute 12,4 g).

Stufe 4:

2-Dimethylaminomethyl-5-trifluormethoxy-indan-1-on

14 g des Produkts aus Stufe 3 wurden in Wasser gelöst, unter Eiskühlung mit 32%iger Natronlauge auf pH 12 gestellt, dreimal mit Dichlormethan extrahiert und die vereinigten organischen Phasen über Magnesiumsulfat getrocknet, filtriert und im Vakuum eingeengt.

Stufe 5:

2-Dimethylaminomethyl-1-pyridin-3-yl-5-trifluormethoxy-indan-1-ol

Zu einer Lösung von 6,0 ml 3-Brompyridin in ca. 108 ml Diethylether p.a. wurden bei einer Temperatur von -35 bis -40 °C 39 ml n-Butyllithiumlösung (1,6 mol/l in n-Hexan) zugetropft. Nach weiteren 20 Minuten bei dieser Temperatur wurden bei fortgesetzter Kühlung 11,3 g des Produkts aus Stufe 4, gelöst in Diethylether p.a. zugetropft, 40 min bei dieser Temperatur weitergerührt und über Nacht bis auf Raumtemperatur erwärmt. Anschließend wurden bei -10 bis 0 °C 41 ml Wasser zugegeben, die Phasen getrennt, die wässrige Phase dreimal mit Diethylether extrahiert (dünnschichtchromatographische Kontrolle), die vereinigten organischen Phasen mit gesättigter Natriumchloridlösung gewaschen und über Magnesiumsulfat getrocknet, filtriert und eingeengt. Das erhaltene Rohprodukt wurde mit Essigsäureethylester/25%iger Ammoniaklösung (V/V = 98:2) an Kieselgel chromatographiert. Es wurden 3,4 g des unpolareren Diastereomeren von 2-Dimethylamino-methyl-1-pyridin-3-yl-5-trifluormethoxy-indan-1-ol sowie 8,7 g einer Mischung des unpolaren und des polaren Diastereomers von 2-Dimethylamino-methyl-1-pyridin-3-yl-5-trifluormethoxy-indan-1-ol, verunreinigt mit Essigsäureamid, erhalten. Das verunreinigte Diastereomerengemisch wurde in 50 ml Diethylether aufgenommen, 20 min bei 40 °C gerührt, unter Eiskühlung weitergerührt und der entstandene Niederschlag abgesaugt. Die Mutterlauge wurde mit 15 ml Diethylether gelöst und der entstandene Niederschlag abgesaugt. Die Mutterlauge wurde in 20 ml Diethyletherln-Hexan (V/V= 1:1) gelöst, über eine MF-Millipore-MCE-Membran (gemischte Celluloseester; 0,45 $\mu$m) filtriert und im Vakuum eingeengt. Es wurden 6,3 g reines Diastereomerengemisch von 2-Dimethylamino-methyl-1-pyridin-3-yl-5-trifluormethoxy-indan-1-ol erhalten, die in ca. 80 ml Aceton p.a gelöst und unter Eiskühlung in jeweils vier Portionen mit ca. 0,322 ml Wasser und ca. 4,54 ml Chlortrimethylsilan in das korrespondierende Hydrochlorid übergeführt, das im Vakuum (ca. 50 mbar) getrocknet wurde (Ausbeute 6,6 g 2-Dimethylamino-methyl-1-pyridin-3-yl-5-trifluormethoxy-indan-1-ol Hydrochlorid (Gemisch der Diastereomeren)).

Hiermit wurde ***Beispiel 20*** 2-Dimethylaminomethyl-1-pyridin-3-yl-5-trifluormethoxy-indan-1-ol Hydrochlorid beschrieben.

***Beispiel 21:***

Dimethyl-(3-pyridin-3-yl-6-trifluormethoxy-1*H*-inden-2-ylmethyl)-amin Hydrochlorid

Zu 1,2 g 2-Dimethylaminomethyl-1-pyridin-3-yl-5-trifluormethoxy-indan-1-ol Hydrochlorid (Gemisch der Diastereomeren) wurden bei Raumtemperatur ca. 10,8 ml 37%ige Salzsäure zugegeben, über Nacht gerührt, 15 ml Aceton p.a. zugegeben und 2 h gerührt. Es bildete sich ein öliger, teilkristalliner Niederschlag, von dem der Überstand abdekantiert wurde. Der Rückstand wurde in ca. 40 ml Aceton p.a. aufgenommen und gerührt, wobei feine Kristalle entstanden, die unter Luftausschluss abgesaugt, zweimal mit Diethylether gewaschen und im Ölpumpenvakuum bei 25 bis 60 °C getrocknet wurden. Es wurden 1,17g Dimethyl-(3-pyridin-3-yl-6-trifluormethoxy-1*H*-inden-2-ylmethyl)-amin Hydrochlorid erhalten.

Hiermit wurde ***Beispiel 21*** Dimethyl-(3-pyridin-3-yl-6-trifluormethoxy-1*H*-inden-2-ylmethyl)-amin Hydrochlorid beschrieben.

***Beispiel 22:***

2-Dimethylaminomethyl-1-pyridin-3-yl-5-trifluormethyl-indan-1-ol Hydrochlorid

Bei Ersatz des Indanons in Beispiel 18 durch 5-Trifluormethyl-indan-1-on und Verwendung von Paraformaldehyd und Dimethylammoniumchlorid in Beispiel 18, Stufe 1, sowie bei Anwendung der im Beispiel 18, Stufen 2 bis 3, beschriebenen Verfahren, wurde die Titelverbindung erhalten.

5-Trifluormethyl-indan-1-on wurde bei Ersatz der 3-(Trifluormethoxy)-zimtsäure in Beispiel 20, Stufe 1, durch 3-(Trifluormethyl)-zimtsäure und unter Anwendung der im Beispiel 20, Stufen 1 bis 2, beschriebenen Verfahren, sowie nachfolgender säulenchromatographischer Abtrennung von 7-Trifluoromethyl-indan-1-on, erhalten.

Hiermit wurde ***Beispiel 22*** 2-Dimethylaminomethyl-1-pyridin-3-yl-5-trifluormethyl-indan-1-ol Hydrochlorid beschrieben.

***Beispiel 24:***

4-Dimethylaminomethyl-5-pyridin-3-yl-2,3,4,5-tetrahydro-benzofbloxepin-5-ol Hydrochlorid

Bei Ersatz des Indanons in Beispiel 18 durch 3,4-Dihydro-2*H*-benzo[*b*]-oxepin-5-on wurde die Titelverbindung

erhalten.

3,4-Dihydro-2*H*-benzo[*b*]oxepin-5-on wurde vorteilhaft in Abweichung von der Literatur (A. Orita, J. Yaruva, J. Otera Angew. Chem. 1999, 111, 2397) durch Reaktion der Phenoxybuttersäure mit Polyphosphorsäure erhalten.

Hiermit wurden die ***Beispiele 24 und 25*** 4-Dimethylaminomethyl-5-pyridin-3-yl-2,3,4,5-tetrahydro-benzo[*b*] oxepin-5-ol Hydrochlorid beschrieben.

***Beispiel 36:***

2-Dimethylaminomethyl-5-methoxy-1-pyridin-3-yl-1,2,3,4-tetrahydronaphthalin-1-ol Hydrochlorid

Stufe 1:

2-Dimethylaminomethyl-5-methoxy-3,4-dihydro-2*H*-naphthalen-1-on Hydrochlorid

Zu einer Lösung von 5 g 5-Methoxy-1-tetralon in 12 ml Acetonitril wurden 3,9 ml Bis-(dimethylamino)-methan gegeben, bei 0 bis 10 °C 2,0 ml Essigsäurechlorid zugetropft, weitere 10 ml Acetonitril hinzugefügt, 3 h bei 50 °C und anschließend 18 h bei Raumtemperatur gerührt. Der entstandene Feststoff wurde abgesaugt, zweimal mit Diethylether gewaschen und im Vakuum getrocknet (Ausbeute 6,4 g).

Stufe 2:

2-Dimethylaminomethyl-5-methoxy-3,4-dihydro-2*H*-naphthalen-1-on

16 g des Produkts aus Stufe 1 wurden in Wasser gelöst, unter Eiskühlung mit 32%iger Natronlauge auf pH 12 gestellt, dreimal mit Dichlormethan extrahiert und die vereinigten organischen Phasen über Magnesiumsulfat getrocknet, filtriert und im Vakuum eingeengt.

Stufe 3:

2-Dimethylaminomethyl-5-methoxy-1-pyridin-3-yl-1,2,3,4-tetrahydronaphthalin-1-ol Hydrochlorid

Zu einer Lösung von 10,0 ml 3-Brompyridin in ca. 180 ml Diethylether p.a. wurden bei einer Temperatur von -35 bis -40 °C ca. 65 ml n-Butyllithiumlösung (1,6 mol/l in n-Hexan) zugetropft. Nach weiteren 20 Minuten bei dieser Temperatur wurden bei fortgesetzter Kühlung 16 g des Produkts aus Stufe 2, gelöst in Diethylether p.a. zugetropft, 40 min bei dieser Temperatur weitergerührt und über Nacht bis auf Raumtemperatur erwärmt.

Anschließend wurden bei -10 bis 0 °C 68 ml Wasser zugegeben, die Phasen getrennt, die wässrige Phase zweimal mit Diethylether extrahiert (dünnschichtchromatographische Kontrolle), die vereinigten organischen Phasen mit gesättigter Natriumchloridlösung gewaschen und über Magnesiumsulfat getrocknet, filtriert und eingeengt.

Das erhaltene Rohprodukt wurde zunächst mit Diethylether/25%Iger Ammoniaklösung (V/V = 98:2) und dann mit Diethylether/Methanol/25%iger Ammoniaklösung (V/V/V = 93:5:2) an Kieselgel chromatographiert.

Es wurden 2,59 g des unpolareren Isomers 2-Dimethylaminomethyl-5-methoxy-1-pyridin-3-yl-1,2,3,4-tetrahydro-naphthalin-1-ol erhalten, die in ca. 100 ml Aceton gelöst und mit ca. 0,075 ml Wasser und ca. 1,05 ml Chlor-trimethylsilan in das korrespondierende Hydrochlorid übergeführt wurden.

Das Hydrochlorid wurde unter Luftausschluss abgesaugt, zweimal mit Diethylether gewaschen und im Vakuum bei Raumtemperatur sowie anschließend im Exsikkator im Ölpumpenvakuum über Sicacide (Schwefelsäure auf inertem silikösem Trägermaterial) getrocknet (Ausbeute 2,4 g).

Es wurden ferner 6,9 g des polareren Isomers von 2-Dimethylaminomethyl-5-methoxy-1-pyridin-3-yl-1,2,3,4-tetrahydronaphthalin-1-ol erhalten, die in ca. 200 ml Aceton gelöst und durch Zugabe von ca. 0,25 ml Wasser und ca. 3,5 ml Chlortrimethylsilan in jeweils drei Portionen und anschließendes Rühren für 1,5 h bei Raumtemperatur in das korrespondierende Hydrochlorid übergeführt wurden. Das Hydrochlorid wurde unter Luftausschluss abgesaugt, zweimal mit Diethylether gewaschen und im Vakuum bei Raumtemperatur sowie anschließend im Exsikkator im Ölpumpenvakuum über Sicacide getrocknet (Ausbeute 7,9 g).

Hiermit wurden die ***Beispiele 36 und 37*** 2-Dimethylaminomethyl-5-methoxy-1-pyridin-3-yl-1,2,3,4-tetrahydronaphthalin-1-ol Hydrochlorid beschrieben

***Beispiel 38:***

(5-Methoxy-1-pyridin-3-yl-3,4-dihydro-naphthalin-2-methyl)-dimethyl-amin Hydrochlorid

Zu 1,2 g des polareren Isomers von 2-Dimethylaminomethyl-5-methoxy-1-pyridin-3-yl-1,2,3,4-tetrahydro-naphthalin-1-ol wurden bei Raumtemperatur ca. 12 ml 37%ige Salzsäure zugegeben, über Nacht bei Raumtemperatur sowie 2 h bei 50 °C gerührt und anschließend bei 70 °C im Vakuum eingeengt. Der Rückstand wurde zweimal in Dichlormethan aufgenommen und wieder eingeengt. Der ölige braune Rückstand wurde in 40 ml Aceton p.a. aufgenommen und gerührt, worauf sich ein kristalliner Feststoff bildete. Es wurde unter Luftausschluss abgesaugt, zweimal mit Diethylether gewaschen und im Ölpumpenvakuum getrocknet.
Der Feststoff wurde nochmals mit 37%iger Salzsäure versetzt und über Nacht bei 45 °C gerührt und bei 70 °C im Vakuum eingeengt. Der Rückstand wurde zweimal in Dichlormethan aufgenommen und wieder eingeengt. Der beigefarbene Feststoff wurde in 40 ml Aceton p.a. aufgenommen und 2 h gerührt, unter Luftausschluss abgesaugt sowie und bei 25 bis 60 °C im Ölpumpenvakuum getrocknet (Ausbeute 0,68 g).

Hiermit wurde ***Beispiel 38*** (5-Methoxy-1-pyridin-3-yl-3,4-dihydro-naphthalin-2-ylmethyl)-dimethyl-amin Hydrochlorid beschrieben.

***Beispiel 39:***

6-Dimethylaminomethyl-5-pyridin-3-yl-7,8-dihydro-naphthalin-1-ol Hydrochlorid

1,25 g des polareren Isomers von 2-Dimethylaminomethyl-5-methoxy-1-pyridin-3-yl-1,2,3,4-tetrahydro-naphthalin-1-ol wurden in ca. 16 ml 33%iger Bromwasserstoffsäure in Essigsäure gelöst und 5 h unter Rückfluss und anschließend 18 h ohne Erhitzen gerührt. Der Reaktionsansatz wurde im Vakuum eingeengt, der Rückstand in Eiswasser aufgenommen und die wässrige Phase mit Diethylether gewaschen. Die wässrige Phase wurde mit 32%iger Natronlauge alkalisch gemacht, mit Essigsäureethylester extrahiert, die vereinigten organischen Phasen über Magnesiumsulfat getrocknet, filtriert und eingeengt.
Das erhaltene Rohprodukt wurde mit Essigsäureethylester/25%iger Ammoniaklösung (V/V = 98:2) an Kieselgel chromatographiert. Es wurden 0,86 g 6-Dimethylaminomethyl-5-pyridin-3-yl-7,8-dihydro-naphthalin-1-ol erhalten, die in ca. 20 ml Aceton p.a. gelöst und unter Eiskühlung mit ca. 0,027 ml Wasser und ca. 0,380 ml Chlortrimethylsilan in das korrespondierende Hydrochlorid übergeführt wurden, das unter Luftausschluss abgesaugt und im Vakuum (ca. 50 mbar) getrocknet wurde (Ausbeute 0,67g).

Hiermit wurde ***Beispiel 39*** 6-Dimethylaminomethyl-5-pyridin-3-yl-7,8-dihydro-naphthalin-1-ol Hydrochlorid beschrieben.

## Pharmakologische Untersuchungen

### a) Methoden zur Bestimmung der Affinität zum humanen μ-Opiatrezeptor und der 5-HT- und NA-Wiederaufnahmehemmung

Untersuchung der Affinität zum humanen μ-Opiatrezeptor

Die Rezeptoraffinität zum humanen μ-Opiatrezeptor wurde in einem homogenen Ansatz in Mikrotiterplatten bestimmt. Hierzu wurden Verdünnungsreihen der zu prüfenden Substanzen mit einer Rezeptormembranpräparation (15 - 40 μg Protein / 250 μl Inkubationsansatz) von CHO-K1-Zellen, welche den humanen μ-Opiatrezeptor exprimieren (RB-HOM-Rezeptormembran-Präparation von Fa. Perkin Elmer, Zaventem, Belgien) in Gegenwart von 1 nmol/l des radioaktiven Liganden [$^3$H]-Naloxon (NET719, Fa. Perkin Eimer, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 μl für 90 Minuten bei Raumtemperatur inkubiert. Als Inkubationspuffer wurden 50 mmol/l Tris-HCl supplementiert mit 50 μM $MgCl_2$ und 0,05 % bovinem Serumalbumin verwendet. Zur Bestimmung der unspezifischen Bindung wurden zusätzlich 100 μmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Fa. PerkinElmer, Freiburg, Deutschland) vermessen. Es wurde die prozentuale Verdrängung des radioaktiven Liganden aus seiner Bindung zum humanen μ-Opiatrezeptor bei einer Konzentration der Prüfsubstanzen von 1 μmol/l bestimmt und als Prozent Hemmung der spezifischen Bindung angegeben. Ausgehend von der prozentualen Verdrängung durch unterschiedliche Konzentrationen der Prüfsubstanzen wurden $IC_{50}$ Hemmkonzentrationen berechnet, die eine 50-prozentige Verdrängung des radioaktiven Liganden bewirken. Durch Umrechnung mittels der Cheng-Prusoff-Beziehung wurden $K_i$-Werte für die Prüfsubstanzen erhalten (Cheng und Prusoff 1973).

Untersuchungen der 5-HT- und NA-Wiederaufnahmehemmung

Um diese in vitro Studien durchführen zu können, wurden Synaptosomen aus Rattenhirnarealen frisch isoliert. Es fand jeweils eine so genannte "$P_2$"-Fraktion Verwendung, die exakt nach der Vorschrift von Gray, E.G. und Whittaker, V.P. (1962, J. Anat. 76, 79-88)) präpariert wurde. Für die NA-Wiederaufnahme wurden diese vesikulären Partikel aus dem Hypothalamus, für die 5-HT- Wiederaufnahme aus der Medulla + Pons-Region von männlichen Rattengehimen isoliert.

Folgende Kenndaten wurden für die NA- und 5-HT- Wiederaufnahme ermittelt:

NA-Uptake: Km = 0,32 $\pm$ 0.11 $\mu$M

5-HT-Uptake : Km = 0,084 $\pm$ 0,011 $\mu$M

(Jeweils N = 4, d.h. Mittelwerte $\pm$ SEM aus 4 unabhängigen Versuchsreihen, die in Dreifach-Parallelversuchen durchgeführt wurden).

Eine detaillierte Methodenbeschreibung ist in der Publikation von Frink, M. Ch., Hennies, H.H., Englberger, W. et al. (1996, Arzneim.-Forsch./Drug Res. 46(III) 11, 1029-1036)) enthalten (der Ansatz kann auch auf Mikrotiterplatten (250 $\mu$l / well) bei Zimmertemperatur durchgeführt werden).

Auswertungen:

Neben % Hemmungen bei fixen Testsubstanzkonzentrationen (z.B. 1 x $10^{-6}$ M oder 1 x $10^{-5}$ M im Ansatz), wurden Dosisabhängigkeiten überprüft. Hierbei wurden $IC_{50}$-Werte erhalten, die gemäß der "Cheng-Prusoff Gleichung" (Cheng, Y.C. und Prusoff, W.H., 1973, Biochem. Pharmacol.22, 3099-3108)) in Inhibitorkonstanten ($K_i$) umgerechnet werden können. Die $IC_{50}$ Werte wurden mit Hilfe des Computer-Programms "Figure P" (Version 6.0, Biosoft, Cambridge, England) erhalten. Km-Werte wurden gemäß Lineweaver, H. und Burk, D. (1934, J. Am. Chem. Soc. 56, 658-666) berechnet. Um $K_D$-werte darzustellen, ist das Computer-Programm "Ligand" (Version 4, Biosoft, England) angewendet worden.

Für die erfindungsgemäßen Verbindungen wurde eine deutliche Affinität zum $\mu$-Opiatrezeptor und/oder eine deutliche Hemmung der Serotonin- und / oder Noradrenalinwiederaufnahme gemessen. Die Ergebnisse von Beispielen sowie für die Referenzsubstanzen Venlafaxin und Duloxetin sind in der nachfolgenden Tabelle dargestellt.

Tabelle 1

| Verbindung gemäß Beispiel Nummer | $\mu$-Opioidrezeptor-Affinität (% Inhibition bei 1 $\mu$mol/l bzw. Ki ($\mu$mol/l)) | 5-HT-Wiederaufnahmehemmung (% Inhibition bei 10 $\mu$mol/l bzw. Ki ($\mu$mol/l)) | NA-Wiederaufnahmehemmung (% Inhibition bei 10 $\mu$mol/l bzw. Ki ($\mu$mol/l)) |
|---|---|---|---|
| 1 | 1 % | 63 % | 33% |
| 2 | 60 % | 89 % | 43 % |
| 3 | 0,24 $\mu$mol/l | 0,013 $\mu$mol/l | 30 % |
| 4 | 31 % | 86 % | 46 % |
| 5 | 49 % | 0,17 $\mu$mol/l | 29 % |
| 6 | 0,15 $\mu$mol/l | 0,028 $\mu$mol/l | 31 % |
| 7 | 0,38 $\mu$mol/l | 0,025 $\mu$mol/l | 59 % |
| 9 | 0,56 $\mu$mol/l | 56 % | 26 % |
| 11 | 20 % | 0,028 $\mu$mol/l | 39 % |
| 12 | 0,38 $\mu$mol/l | 0,12 $\mu$mol/l | 20 % |
| 13 | - 23 % | 41 % | 45 % |
| 14 | -14 % | 44 % | 54 % |
| 15 | 0,32 $\mu$mol/l | 60 % | 36 % |
| 16 | 0,21 $\mu$mol/l | 35 % | -2 % |

(fortgesetzt)

| Verbindung gemäß Beispiel Nummer | μ-Opioidrezeptor-Affinität (% Inhibition bei 1 μmol/l bzw. Ki (μmol/l)) | 5-HT-Wiederaufnahmehemmung (% Inhibition bei 10 μmol/l bzw. Ki (μmol/l)) | NA-Wiederaufnahmehemmung (% Inhibition bei 10 μmol/l bzw. Ki (μmol/l)) |
|---|---|---|---|
| 17 | -13% | 57% | 19% |
| 18 | -6% | 56% | 29% |
| 19 | 0,29 μmol/l | 0,043 μmol/l | 31 % |
| 20 | -19 % | 71 % | 38 % |
| 21 | 5 % | 0,43 μmol/l | 14 % |
| 22 | 2% | 61 % | 17% |
| 23 | -8% | 47% | 6% |
| 24 | 45% | 12% | 9% |
| 26 | 37% | 50 % | 9% |
| 31 | - | -4% | 48% |
| 36 | -14% | 33% | 49% |
| 37 | 8% | 19% | 57% |
| 38 | 0,46 μmol/l | 0,37 μmol/l | 34% |
| 39 | 0,02 μmol/l | 0,25 μmol/l | 50 % |
| Venlafaxin | -1 % | 0,062 μmol/l | 0,45 μmol/l |
| Duloxetin | -10 % | 0,0046 μmol/l | 0,057 μmol/l |

**a) Untersuchungen der analgetischen Wirkungen im Formalin-Test an der Maus und an der Ratte**

Der Formalin Test (Dubuisson, D. and Dennis, S.G., 1977, Pain, 4,161 - 174) stellt ein Modell für den akuten sowie den chronischen Schmerz dar. Durch eine einmalige Formalin-Injektion in die dorsale Seite einer Hinterpfote wurde bei freibeweglichen Versuchstieren eine biphasige nozizeptive Reaktion induziert, die durch Beobachtung von drei deutlich voneinander unterscheidbaren Verhaltensmustern erfasst wurde. Die Reaktion ist zweiphasig: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input bzw. Glutamatfreisetzung (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalintest an der Ratte:

Formalin wurde mit dem Volumen von 50μl und einer Konzentration von 5 % subkutan in die dorsale Seite der rechten Hinterpfote jedes Tieres appliziert. Die spezifischen Verhaltensänderungen, wie Anheben und Schütteln der Pfote, Gewichtsverlagerungen des Tieres sowie Beiß- und Leckreaktionen wurden im Beobachtungszeitraum von 21 bis 27 min nach Formalininjektion beobachtet und registriert. Die Zusammenfassung der verschiedenen Verhaltensweisen erfolgte in der sogenannten Pain-Rate (PR), die, auf die Teilintervalle von 3 min bezogen, die Berechnung einer mittleren Nociceptionsreaktion darstellt. Die Berechnung der PR erfolgte aufgrund einer numerischen Gewichtung (= jeweils Faktor 1, 2, 3) der beobachteten Verhaltensweisen (entsprechend Verhaltensscore 1, 2, 3) und wurde mit folgender Formel berechnet:

$$PR = [(T_0 \times 0) + (T_1 \times 1) + (T_2 \times 2) + (T_3 \times 3)] / 180,$$

wobei $T_0$, $T_1$, $T_2$, und $T_3$ jeweils der Zeit in Sekunden entspricht, in der das Tier die Verhaltensweisen 0, 1, 2 oder 3 zeigte. Die Gruppengröße betrug 10 Tiere (n=10).

Formalintest an der Maus:

Formalin wurde mit dem Volumen von 20µl und einer Konzentration von 1 % subkutan in die dorsale Seite der rechten Hinterpfote jedes Tieres appliziert. Die spezifischen Verhaltensänderungen, wie Anheben und Schütteln der Pfote (score 3), wurden im Beobachtungszeitraum von 21 bis 24 min nach Formalininjektion beobachtet und registriert. Die Gruppengröße betrug 10 Tiere (n=10).

Basierend auf den PR-Berechnungen wurde die Substanzwirkung als Änderung gegen eine Kontrolle in Prozent ermittelt. Die $ED_{50}$-Bestimmung erfolgte mittels Regressionsanalyse.

Für die erfindungsgemäßen Verbindungen sowie für die Referenzsubstanzen Venlafaxin und Duloxetin wurde eine Hemmung des nociceptiven Verhaltens beobachtet. Die Ergebnisse sind in der nachfolgenden Tabelle dargestellt.

Tabelle 2

| **Verbindung gemäß Beispiel Nummer** | **Spezies** | **Dosis (mg/kg i.v.)** | **Hemmung des nociceptiven Verhaltens versus Kontrolle (% Hemmung)** | **Hemmung des nociceptiven Verhaltens versus Kontrolle, $ED_{50}$- Wert (mg/kg i.v.)** |
|---|---|---|---|---|
| 2 | Ratte | 46,4 | -80,6 | - |
| 3 | Maus | - | - | 3,32 |
| 4 | Ratte | 4,64 | -61,60 | |
| 5 | Ratte | 21,5 | -43,0 | |
| 6 | Maus | - | - | 1,64 |
| 7 | Ratte | - | - | 2,93 |
| 8 | Ratte | 46,4 | -65,8 | - |
| 9 | Ratte | 10 | -97,0 | |
| 12 | Maus | 14,7 | -45,8 | - |
| 13 | Ratte | 21,5 | -53,6 | - |
| 14 | Ratte | 21,5 | -53,2 | - |
| 15 | Ratte | 10,0 | -70,7 | - |
| Venlafaxin | Maus | - | - | 2,60 |
| Duloxetin | Ratte | - | - | 1,43 |

**Patentansprüche**

**1.** Gesättigte und ungesättigte 3-Pyridyl-benzocycloalkylmethyl-amine der allgemeinen Formel I

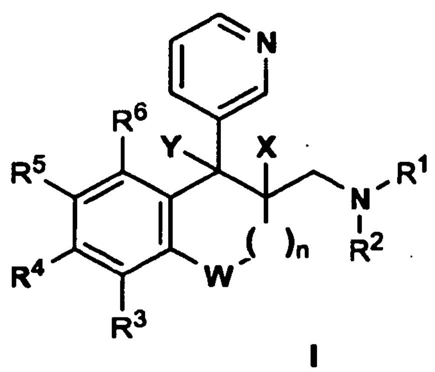

I

worin
W gleich $CH_2$, O, S, SO oder $SO_2$ ist und n = 0 - 3 ist,
$R^1$ und $R^2$ unabhängig voneinander ausgewählt sind aus H und $C_1$-$C_{10}$-Alkyl, jeweils verzweigt oder unverzweigt, einfach oder mehrfach substituiert oder unsubstituiert;
und
$R^3$ bis $R^6$ unabhängig voneinander aus H oder beliebigen Resten mit Ausnahme von weiteren ankondensierten Ringen ausgewählt sind,
wobei man unter diesen Resten F, Cl, Br, I, CN, $CF_3$, $OCF_3$, $C_1$-$C_{10}$-Alkyl, jeweils verzweigt oder unverzweigt, einfach oder mehrfach substituiert oder unsubstituiert versteht;
$OR^{6'}$ oder $SR^{6'}$ wobei $R^{6'}$ ausgewählt ist aus H und $C_1$-$C_{10}$-Alkyl, jeweils verzweigt oder unverzweigt, einfach oder mehrfach substituiert oder unsubstituiert versteht;
und Y ausgewählt ist aus H oder OH, wenn gleichzeitig X gleich H ist, oder X und Y zusammen eine Bindung bilden, wobei unter "substituiert" die Substitution eines Wasserstoffrestes durch F, Cl, Br, I, $NH_2$, SH, OH oder $OCH_3$ verstanden wird,
auch in Form ihrer Razemate, Enantiomere, Diastereomere, insbesondere Mischungen ihrer Enantiomere oder Diastereomere oder eines einzelnen Enantiomers oder Diastereomers sowie ihrer freien Basen oder eines mit einer physiologisch verträglichen Säure gebildeten Salzes.

2. Verbindungen der allgemeinen Formel I nach Anspruch 1, **dadurch gekennzeichnet, dass** n < 3 ist.

3. Verbindungen der allgemeinen Formel I nach Anspruch 1 oder 2, **dadurch gekennzeichnet, dass** W = $CH_2$ ist und n < 3 ist.

4. Verbindungen der allgemeinen Formel I nach einem der Ansprüche 1 bis 3, **dadurch gekennzeichnet, dass** W = $CH_2$ ist, n < 3 ist, $R^1$ und $R^2$ = Methyl sind und $R^5$ und $R^6$ = H bedeuten.

5. Verbindungen der allgemeinen Formel I nach Anspruch 1 oder 2, wobei die Verbindungen ausgewählt sind aus:

2-Dimethylaminomethyl-1-pyridin-3-yl-indan-1-ol und das entsprechende Hydrochlorid (polares Diastereomer) **(1)**
2-Dimethylaminomethyl-1-pyridin-3-yl-indan-1-ol und das entsprechende Hydrochlorid (unpolares Diastereomer) **(2)**
Dimethyl-(3-pyridin-3-yl-1*H*-inden-2-ylmethyl)-amin und das entsprechende Hydrochlorid **(3)**
Dimethyl-(1-pyridin-3-yl-indan-2-ylmethyl)-amin und das entsprechende Hydrochlorid **(4)**
2-Dimethylaminomethyl-5-methoxy-1-pyridin-3-yl-indan-1-ol und das entsprechende Hydrochlorid (polares und unpolares Diastereomer) **(5)**
(6-Methoxy-3-pyridin-3-yl-1*H*-inden-2-ylmethyl)-dimethyl-amin und das entsprechende Hydrochlorid **(6)**
2-Dimethylaminomethyl-1-pyridin-3-yl-3*H*-inden-5-ol und das entsprechende Hydrochlorid **(7)**
2-Dimethylaminomethyl-1-pyridin-3-yl-1,2,3,4-tetrahydro-naphthalin-1-ol und das entsprechende Hydrochlorid (polares und unpolares Diastereomer) **(8)**
Dimethyl-(1-pyridin-3-yl-3,4-dihydro-naphthalin-2-ylmethyl)-amin und das entsprechende Hydrochlorid **(9)**
Dimethyl-(1-pyridin-3-yl-1,2,3,4-tetrahydro-naphthalin-2-ylmethyl)-amin und das entsprechende Hydrochlorid **(10)**
6-Dimethylaminomethyl-5-pyridin-3-yl-6,7,8,9-tetrahydro-5*H*-benzocyclohepten-5-ol und das entsprechende Hydrochlorid **(11)**
Dimethyl-(5-pyridin-3-yl-8,9-dihydro-7*H*-benzocyclohepten-6-ylmethyl)-amin und das entsprechende Hydrochlorid **(12)**
4-Dimethylaminomethyl-5-pyridin-3-yl-2,3,4,5-tetrahydro-benzo[b]thiepin-5-ol und das entsprechende Hydrochlorid (polares Diastereomer) **(13)**
4-Dimethylaminomethyl-5-pyridin-3-yl-2,3,4,5-tetrahydro-benzo[b]thiepin-5-ol und das entsprechende Hydrochlorid (unpolares Diastereomer) **(14)**
Dimethyl-(5-pyridin-3-yl-2,3-dihydro-benzo[b]thiepin-4-ylmethyl)-amin und das entsprechende Hydrochlorid **(15)**
Dimethyl-(1-oxo-5-pyridin-3-yl-2,3-dihydro-1*H*-1λ4-benzo[b]thiepin-4-ylmethyl)-amin und das entsprechende Hydrochlorid **(16)**
4-Dimethylaminomethyl-1,1-dioxo-5-pyridin-3-yl-2,3,4,5-tetrahydro-1*H*-1λ6-benzo[*b*]thiepin-5-ol und das entsprechende Hydrochlorid **(17)**
2-Dimethylaminomethyl-5-fluor-1-pyridin-3-yl-indan-1-ol und das entsprechende Hydrochlorid (polares und unpolares Diastereomer) **(18)**
(6-Fluor-3-pyridin-3-yl-1*H*-inden-2-ylmethyl-dimethyl-amin und das entsprechende Hydrochlorid **(19)**
2-Dimethylaminomethyl-1-pyridin-3-yl-5-trifluormethoxy-indan-1-ol und das entsprechende Hydrochlorid (polares und unpolares Diastereomer) **(20)**
Dimethyl-(3-pyridin-3-yl-6-trifluormethoxy-1*H*-inden-2-ylmethyl)-amin und das entsprechende Hydrochlorid **(21)**
2-Dimethylaminomethyl-1-pyridin-3-yl-5-trifluormethyl-indan-1-ol und das entsprechende Hydrochlorid (polares und unpolares Diastereomer) **(22)**
Dimethyl-(3-pyridin-3-yl-6-trifluormethyl-1*H*-inden-2-ylmethyl)-amin und das entsprechende Hydrochlorid **(23)**
4-Dimethylaminomethyl-5-pyridin-3-yl-2,3,4,5-tetrahydro-benzo[*b*]oxepin-5-ol und das entsprechende Hydrochlorid (unpolares Diastereomer) **(24)**
4-Dimethylaminomethyl-5-pyridin-3-yl-2,3,4,5-tetrahydro-benzo[*b*]oxepin-5-ol und das entsprechende Hydrochlorid (polares Diastereomer) **(25)**
Dimethyl-(5-pyridin-3-yl-2,3-dihydro-benzo[*b*]oxepin-4-ylmethyl)-amin und das entsprechende Hydrochlorid **(26)**
3-Dimethylaminomethyl-4-pyridin-3-yl-chroman-4-ol und das entsprechende Hydrochlorid (unpolares Diastereomer) **(27)**
3-Dimethylaminomethyl-4-pyridin-3-yl-chroman-4-ol und das entsprechende Hydrochlorid (polares Diastereomer) **(28)**
Dimethyl-(4-pyridin-3-yl-2*H*-chromen-3-ylmethyl)-amin und das entsprechende Hydrochlorid **(29)**
3-Dimethylaminomethyl-4-pyridin-3-yl-thiochroman-4-ol und das entsprechende Hydrochlorid (unpolares Diastereomer) **(30)**
3-Dimethylaminomethyl-4-pyridin-3-yl-thiochroman-4-ol und das entsprechende Hydrochlorid (polares Diastereomer) **(31)**
Dimethyl-(4-pyridin-3-yl-2*H*-thiochromen-3-ylmethyl)-amin und das entsprechende Hydrochlorid **(32)**
2-Dimethylaminomethyl-6-methoxy-1-pyridin-3-yl-indan-1-ol und das entsprechende Hydrochlorid (unpolares Diastereomer) **(33)**
2-Dimethylaminomethyl-6-methoxy-1-pyridin-3-yl-indan-1-ol und das entsprechende Hydrochlorid (polares Diastereomer) **(34)**
(5-Methoxy-3-pyridin-3-yl-1*H*-inden-2-ylmethyl)-dimethyl-amin und das entsprechende Hydrochlorid **(35)**
2-Dimethylaminomethyl-5-methoxy-1-pyridin-3-yl-1,2,3,4-tetrahydronaphthalin-1-ol und das entsprechende Hydrochlorid (unpolares Diastereomer) **(36)**
2-Dimethylaminomethyl-5-methoxy-1-pyridin-3-yl-1,2,3,4-tetrahydronaphthalin-1-ol und das entsprechende Hydrochlorid (polares Diastereomer) **(37)**
(5-Methoxy-1-pyridin-3-yl-3,4-dihydro-naphthalin-2-ylmethyl)-dimethyl-amin und das entsprechende Hydrochlorid **(38)**
6-Dimethylaminomethyl-5-pyridin-3-yl-7,8-dihydro-naphthalin-1-ol und das entsprechende Hydrochlorid **(39).**

**6.** Verbindungen nach Anspruch 1, 2 oder 5, **dadurch gekennzeichnet, dass** sie ausgewählt sind aus:

Dimethyl-(3-pyridin-3-yl-1*H*-inden-2-ylmethyl)-amin und das entsprechende Hydrochlorid **(3)**
(6-Methoxy-3-pyridin-3-yl-1*H*-inden-2-ylmethyl)-dimethyl-amin und das entsprechende Hydrochlorid **(6)**
2-Dimethylaminomethyl-1-pyridin-3-yl-3*H*-inden-5-ol und das entsprechende Hydrochlorid **(7)**
6-Dimethylaminomethyl-5-pyridin-3-yl-6,7,8,9-tetrahydro-5*H*-benzocyclohepten-5-ol und das entsprechende Hydrochlorid **(11)**
Dimethyl-(5-pyridin-3-yl-8,9-dihydro-7*H*-benzocyclohepten-6-ylmethyl)-amin und das entsprechende Hydrochlorid **(12)**
(6-Fluor-3-pyridin-3-yl-1*H*-inden-2-ylmethyl)-dimethyl-amin und das entsprechende Hydrochlorid **(19)**
Dimethyl-(3-pyridin-3-yl-6-trifluormethoxy-1*H*-inden-2-ylmethyl)-amin und das entsprechende Hydrochlorid **(21)**
(5-Methoxy-1-pyridin-3-yl-3,4-dihydro-naphthalin-2-ylmethyl-dimethyl-amin und das entsprechende Hydrochlorid **(38)**
6-Dimethylaminomethyl-5-pyridin-3-yl-7,8-dihydro-naphthalin-1-ol und das entsprechende Hydrochlorid **(39).**

**7.** Verfahren zur Herstellung von Verbindungen der allgemeinen Formel **I** sowie ihrer pharmazeutisch annehmbaren Salze, **dadurch gekennzeichnet, dass** Cycloalkanone der allgemeinen Formel **II**

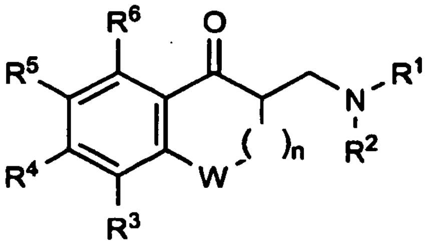

II

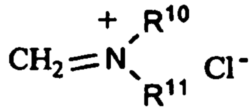

$NHR^{10}R^{11}$

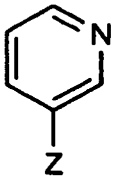

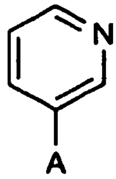

III IV V VI

mit Immoniumsalzen der Formel **III** bzw. mit Paraformaldehyd und einem Amin der Formel **IV** umgesetzt werden, wobei $R^{10}$ eine $R^1$ analoge Bedeutung und $R^{11}$ eine $R^2$ analoge Bedeutung hat, die erhaltenen Mannich-Basen mit einer metallorganischen Verbindung der Formel **V,** in der **Z** Li bedeutet, in Lösungsmittel zwischen -70°C und 60°C umgesetzt werden, wobei im Falle, dass entweder $R^{10}$ oder $R^{11}$ oder beide gleichzeitig gleich Wasserstoff sind, in die Mannich-Reaktion Verbindungen der allgemeinen Formel **III** bzw. **IV** eingesetzt werden, in denen $R^{10}$ oder $R^{11}$ oder $R^{10}$ und $R^{11}$ einen Benzylrest darstellt, welcher durch katalytische Umsetzung mit Wasserstoff entfernt wird, und wobei Verbindungen der Formel **V,** in der **Z** Lithium oder Magnesium-Halogenid bedeutet, durch Umsetzung von Halogenverbindungen der Formel **VI,** in der **A** gleich **Cl**, Br oder **I** bedeutet, mit vorzugsweise n-Butyllithium/ Hexan-Lösung durch Halogen-Lithiumaustausch hergestellt werden, oder Verbindungen der Formel **V** in Gegenwart von Cer(II)halogenid mit Verbindungen der Formel **II** umgesetzt werden, wodurch Produkte der allgemeinen Formel **VII,**

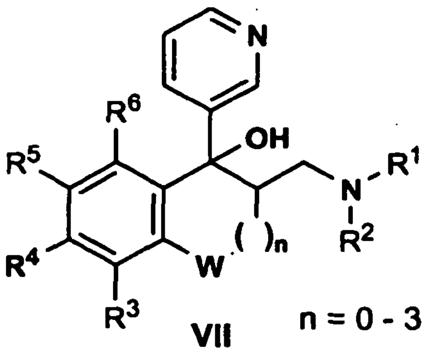

VII n = 0 - 3

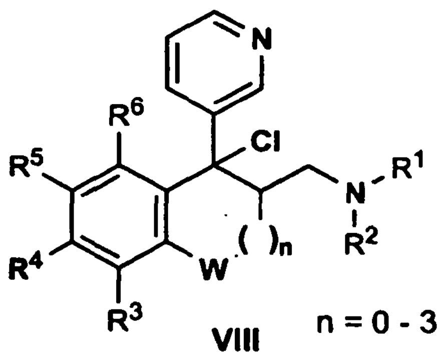

VIII n = 0 - 3

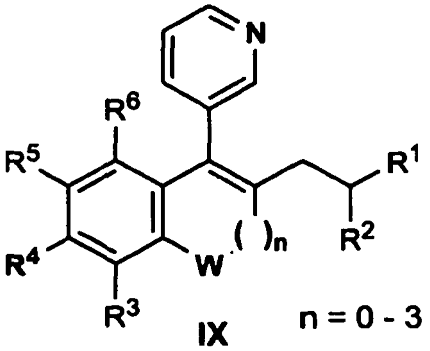

IX n = 0 - 3

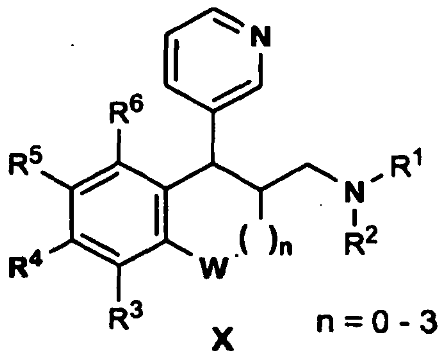

X n = 0 - 3

in der $R^{10}$ eine $R^1$ analoge Bedeutung und $R^{11}$ eine $R^2$ analoge Bedeutung hat, erhalten werden, die mit Thionylchlorid umgesetzt und anschließend basisch zu Verbindungen der allgemeinen Formel **IX** oder einem Gemisch aus Verbindungen der allgemeinen Formeln **VIII** und **IX,** in denen $R^{10}$ eine $R^1$ analoge und $R^{11}$ eine $R^2$ analoge Bedeutung hat, aufgearbeitet werden, die Verbindungen getrennt werden, oder die Verbindungen der allgemeinen Formel **IX** durch Umsetzen von Verbindungen der allgemeinen Formel **VII** mit starken Säuren hergestellt werden, anschließend die hydrogenolytische Spaltung von Verbindungen der allgemeinen Formel **VIII** oder die Hydrierung von Verbindungen der allgemeinen Formel **IX,** in der $R^{10}$ eine $R^1$ analoge Bedeutung und $R^{11}$ eine $R^2$ analoge Bedeutung hat, durch katalytische Reaktion mit Wasserstoff zu Verbindungen der Formel **X,** in der $R^{10}$ eine $R^1$ analoge Bedeutung und $R^{11}$ eine $R^2$ analoge Bedeutung hat, in Lösungsmittel bei Drücken von 0,1 bis 10 bar und Temperaturen von 20°C bis 80°C durchgeführt wird, wobei Verbindungen der allgemeinen Formel **I** mit W gleich SO bzw. $SO_2$ aus entsprechenden Verbindungen mit W gleich S durch Oxidation hergestellt werden.

**8.** Verfahren zur Herstellung von ungesättigten 3-Pyridyl-benzocycloalkylmethylaminen der allgemeinen Formel **I,** in der X und Y zusammen eine Bindung bilden, **dadurch gekennzeichnet, dass** eine Übergangsmetall-katalysierte Kreuzkopplung von Verbindungen der allgemeinen Formel **XI,**

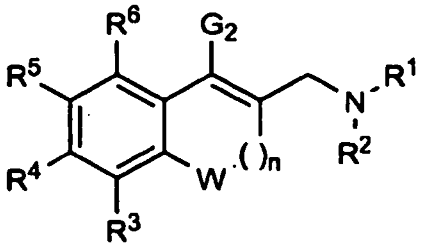

**XI**

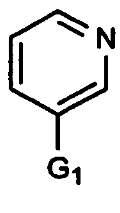

**XII**

in der $G_2$ gleich Cl, Br, I, $Sn(Alkyl)_3$ oder $OSO_2CF_3$ bedeutet, mit Verbindungen der allgemeinen Formel **XII,** in der $G_1$ gleich Cl, Br, I oder $B(OR^x)_2$ bedeutet, mit $R^x$ ausgewählt aus H oder Alkyl, wobei die Reaktion mit oder ohne Base und mit oder ohne Ligand durchgeführt werden kann, erfolgt.

**9.** Verfahren zur Herstellung von ungesättigten 3-Pyridyl-benzocycloalkylmethylaminen der allgemeinen Formel **I,** in der X und Y zusammen eine Bindung bilden und W gleich O oder S sowie n = 1 ist, **dadurch gekennzeichnet, dass** ortho-halogensubstituierte Phenole, wobei Halogen ausgewählt ist aus Br, I, $OSO_2CF_3$, /Thiophenote der allgemeinen Formel **XIII**

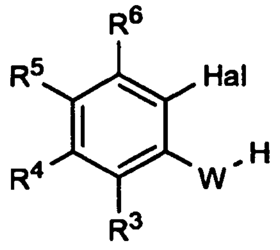

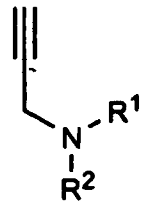

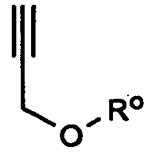

**XIII** **XIV** **XV**

mit substituierten Propargylaminen der allgemeinen Formel **XIV** oder mit Propargylalkoholen oder deren Derivaten der allgemeinen Formel **XV** mit R° ausgewählt aus **H** oder einer Schutzgruppe umgesetzt werden, wobei im Fall der Propargylalkohole oder deren Derivate der allgemeinen Formel **XV** die Funktion OR° in einem nachgeschalteten Reaktionsschritt in an sich bekannter Weise in die Aminofunktion $NR^1R^2$ übergeführt wird.

**10.** Verfahren nach einem der Ansprüche 7, 8 oder 9, **dadurch gekennzeichnet, dass** mindestens eine in Formel **I** enthaltene OH-Gruppe durch eine $OSi(Ph)_2$tert.but.-Gruppe, mindestens eine SH-Gruppe durch eine S-p-Methoxybenzylgruppe und /oder mindestens eine $NH_2$-Gruppe durch eine $NO_2$-Gruppe ersetzt wird und nach Abschluss der gesamten Reaktionssequenz eine $OSi(Ph)_2$tert.but.-Gruppe mit Tetrabutylammoniumfluorid in Tetrahydrofuran und/oder mindestens eine p-Methoxybenzylgruppe mit einem Metallamin, bevorzugt Natriumamin, abgespalten und/oder mindestens eine $NO_2$-Gruppe zu $NH_2$ reduziert wird.

**11.** Arzneimittel, enthaltend mindestens eine Verbindung der allgemeinen Formel **I** oder eines ihrer pharmazeutisch annehmbaren Salze.

**12.** Verwendung einer Verbindung der allgemeinen Formel **I** oder eines ihrer pharmazeutisch annehmbaren Salze zur Herstellung eines Arzneimittels zur Behandlung von Schmerzen, Depressionen und/oder Angststörungen.

**13.** Verwendung einer Verbindung der allgemeinen Formel **I** oder eines ihrer pharmazeutisch annehmbaren Salze zur Herstellung eines Arzneimittels zur Behandlung von Migräne, Harninkontinenz, Fibromyalgia, Essstörungen, Bulimie, Hyperaktivität, Drogenabhängigkeit, -sucht und -entzug, Trichotillomanie, Tourette's Syndrom, Hauterkrankungen wie Postherpetische Neuralgie und Pruritus, Psychosen, Gedächtnisstörungen, kognitiven Störungen und/oder Morbus Alzheimer.

**14.** Pharmazeutische Zusammensetzung, die mindestens eine Verbindung der allgemeinen Formel **I** oder eines ihrer pharmazeutisch annehmbaren Salze sowie mindestens einen pharmazeutischen Hilfsstoff enthält.

## Claims

**1.** Saturated and unsaturated 3-pyridyl benzocycloalkylmethylamines of the general formula **I**

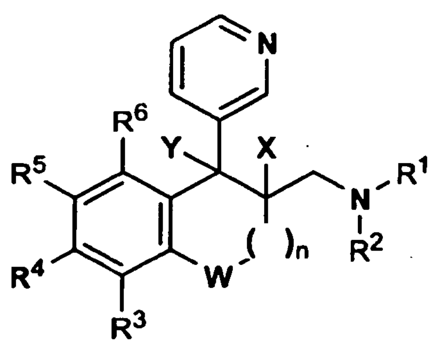

**I** ,

in which
W is $CH_2$, O, S, SO or $SO_2$ and n is 0-3,
$R^1$ and $R^2$ are mutually independently selected from H and $C_1$-$C_{10}$ alkyl, in each case branched or unbranched, mono- or polysubstituted or unsubstituted;
and
$R^3$ to $R^6$ are mutually independently selected from H or any desired residues with the exception of further fused rings, these residues being taken to be F, Cl, Br, I, CN, $CF_3$, $OCF_3$, $C_1$-$C_{10}$ alkyl, in each case branched or unbranched, mono- or polysubstituted or unsubstituted;
$OR^{6'}$ or $SR^{6'}$, $R^{6'}$ being selected from H and $C_1$-$C_{10}$ alkyl, in each case branched or unbranched, mono- or polysubstituted or unsubstituted;
and Y is selected from H or OH, if X is simultaneously H, or X and Y together form a bond,
"substituted" being taken to mean substitution of a hydrogen residue by F, Cl, Br, I, $NH_2$, SH, OH or $-OCH_3$,
also in the form of the racemates, enantiomers, diastereomers thereof, in particular mixtures of the enantiomers or diastereomers thereof or of an individual enantiomer or diastereomer and the free bases thereof or of a salt formed with a physiologically acceptable acid.

2. Compounds of the general formula **I** according to claim 1, **characterised in that** n is < 3.

3. Compounds of the general formula **I** according to claim 1 or claim 2, **characterised in that** W is $CH_2$ and n is < 3.

4. Compounds of the general formula **I** according to any one of claims 1 to 3, **characterised in that** W is $CH_2$, n is < 3, $R^1$ and $R^2$ are methyl and $R^5$ and $R^6$ mean H.

5. Compounds of the general formula **I** according to claim 1 or claim 2, the compounds being selected from:

2-dimethylaminomethyl-1-pyridin-3-ylindan-1-ol and the corresponding hydrochloride (polar diastereomer) **(1)**
2-dimethylaminomethyl-1-pyridin-3-ylindan-1-ol and the corresponding hydrochloride (nonpolar diastereomer) **(2)**
dimethyl-(3-pyridin-3-yl-1*H*-inden-2-ylmethyl)amine and the corresponding hydrochloride **(3)**
dimethyl-(1-pyridin-3-ylindan-2-ylmethyl)amine and the corresponding hydrochloride **(4)**
2-dimethylaminomethyl-5-methoxy-1-pyridin-3-ylindan-1-ol and the corresponding hydrochloride (polar and nonpolar diastereomer) **(5)**
(6-methoxy-(3-pyridin-3-yl-1*H*-inden-2-ylmethyl)dimethylamine and the corresponding hydrochloride **(6)**
2-dimethylaminomethyl-1-pyridin-3-yl-3H-inden-5-ol and the corresponding hydrochloride **(7)**
2-dimethylaminomethyl-1-pyridin-3-yl-1,2,3,4-tetrahydronaphthalen-1-ol and the corresponding hydrochloride (polar and nonpolar diastereomer) **(8)**
dimethyl-(1-pyridin-3-yl-3,4-dihydronaphthalen-2-ylmethyl)amine and the corresponding hydrochloride **(9)**
dimethyl-(1-pyridin-3-yl-1,2,3,4-tetrahydronaphthalene-2-ylmethyl)amine and the corresponding hydrochloride **(10)**
6-dimethylaminomethyl-5-pyridin-3-yl-6,7,8,9-tetrahydro-5*H*-benzocyclohepten-5-ol and the corresponding hydrochloride **(11)**
dimethyl-(5-pyridin-3-yl-8,9-dihydro-7H-benzocyclohepten-6-ylmethyl)amine and the corresponding hydrochloride **(12)**
4-dimethylaminomethyl-5-pyridin-3-yl-2,3,4,5-tetrahydrobenzo[*b*]thiepin-5-ol and the corresponding hydrochloride (polar diastereomer) **(13)**
4-dimethylaminomethyl-5-pyridin-3-yl-2,3,4,5-tetrahydrobenzo[*b*]thiepin-5-ol and the corresponding hydrochloride (nonpolar diastereomer) **(14)**
dimethyl-(5-pyridin-3-yl-2,3-dihydrobenzo[*b*]thiepin-4-ylmethyl)amine and the corresponding hydrochloride **(15)**
dimethyl)-(1-oxo-5-pyridin-3-yl-2,3-dihydro-1*H*-1λ4-benzo[*b*]thiepin-4-ylmethyl)amine and the corresponding hydrochloride **(16)**
4-dimethylaminomethyl-1,1-dioxo-5-pyridin-3-yl-2,3,4,5-tetrahydro-1*H*-1λ6-benzo[*b*]thiepin-5-ol and the corresponding hydrochloride **(17)**
2-dimethylaminomethyl-5-fluoro-1-pyridin-3-ylindan-1-ol and the corresponding hydrochloride (polar and nonpolar diastereomer) **(18)**
(6-fluoro-3-pyridin-3-yl-1*H*-inden-2-ylmethyl)dimethylamine and the corresponding hydrochloride **(19)**
2-dimethylaminomethyl-1-pyridin-3-yl-5-trifluoromethoxyindan-1-ol and the corresponding hydrochloride (polar and nonpolar diastereomer) **(20)**
dimethyl-(3-pyridin-3-yl-6-trifluoromethoxy-1*H*-inden-2-ylmethyl)amine and the corresponding hydrochloride

**(21)**
2-dimethylaminomethyl-1-pyridin-3-yl-5-trifluoromethylindan-1-ol and the corresponding hydrochloride (polar and nonpolar diastereomer) (22)
dimethyl-(3-pyridin-3-yl-6-trifluoromethyl-1*H*-inden-2-ylmethyl)amine and the corresponding hydrochloride **(23)**
4-dimethylaminomethyl-5-pyridin-3-yl-2,3,4,5-tetrahydrobenzo[*b*]oxepin-5-ol and the corresponding hydrochloride (nonpolar diastereomer) **(24)**
4-dimethylaminomethyl-5-pyridin-3-yl-2,3,4,5-tetrahydrobenzo[*b*]oxepin-5-ol and the corresponding hydrochloride (polar diastereomer) **(25)**
dimethyl-(5-pyridin-3-yl-2,3-dihydrobenzo[*b*]oxepin-4-ylmethyl)amine and the corresponding hydrochloride **(26)**
3-dimethylaminomethyl-4-pyridin-3-ylchroman-4-ol and the corresponding hydrochloride (nonpolar diastereomer) **(27)**
3-dimethylaminomethyl-4-pyridin-3-ylchroman-4-ol and the corresponding hydrochloride (polar diastereomer) **(28)**
dimethyl-(4-pyridin-3-yl-2*H*-chromen-3-ylmethyl)amine and the corresponding hydrochloride **(29)**
3-dimethylaminomethyl-4-pyridin-3-ylthiochroman-4-ol and the corresponding hydrochloride (nonpolar diastereomer) **(30)**
3-dimethylaminomethyl-4-pyridin-3-ylthiochroman-4-ol and the corresponding hydrochloride (polar diastereomer) **(31)**
dimethyl-(4-pyridin-3-yl-2*H*-thiochromen-3-ylmethyl)amine and the corresponding hydrochloride **(32)**
2-dimethylaminomethyl-6-methoxy-1-pyridin-3-ylindan-1-ol and the corresponding hydrochloride (nonpolar diastereomer) **(33)**
2-dimethylaminomethyl-6-methoxy-1-pyridin-3-ylindan-1-ol and the corresponding hydrochloride (polar diastereomer) **(34)**
(5-methoxy-(3-pyridin-3-yl-1*H*-inden-2-ylmethyl)dimethylamine and the corresponding hydrochloride **(35)**
2-dimethylaminomethyl-5-methoxy-1-pyridin-3-yl-1,2,3,4-tetrahydronaphthalen-1-ol and the corresponding hydrochloride (nonpolar diastereomer) **(36)**
2-dimethylaminomethyl-5-methoxy-1-pyridin-3-yl-1,2,3,4-tetrahydronaphthalen-1-ol and the corresponding hydrochloride (polar diastereomer) **(37)**
(5-methoxy-1-pyridin-3-yl-3,4-dihydronaphthalen-2-ylmethyl)dimethylamine and the corresponding hydrochloride **(38)**
6-dimethylaminomethyl-5-pyridin-3-yl-7,8-dihydronaphthalen-1-ol and the corresponding hydrochloride **(39).**

**6.** Compounds according to claim 1, 2 or 5, **characterised in that** they are selected from:

dimethyl-(3-pyridin-3-yl-1*H*-inden-2-ylmethyl)amine and the corresponding hydrochloride **(3)**
(6-methoxy-(3-pyridin-3-yl-1*H*-inden-2-ylmethyl)dimethylamine and the corresponding hydrochloride **(6)**
2-dimethylaminomethyl-1-pyridin-3-yl-3H-inden-5-ol and the corresponding hydrochloride **(7)**
6-dimethylaminomethyl-5-pyridin-3-yl-6,7,8,9-tetrahydro-5*H*-benzocyclohepten-5-ol and the corresponding hydrochloride **(11)**
dimethyl-(5-pyridin-3-yl-8,9-dihydro-7H-benzocyclohepten-6-ylmethyl)amine and the corresponding hydrochloride **(12)**
(6-fluoro-3-pyridin-3-yl-1*H*-inden-2-ylmethyl)dimethylamine and the corresponding hydrochloride **(19)**
dimethyl-(3-pyridin-3-yl-6-trifluoromethoxy-1*H*-inden-2-ylmethyl)amine and the corresponding hydrochloride **(21)**
(5-methoxy-1-pyridin-3-yl-3,4-dihydronaphthalen-2-ylmethyl)dimethylamine and the corresponding hydrochloride **(38)**
6-dimethylaminomethyl-5-pyridin-3-yl-7,8-dihydronaphthalen-1-ol and the corresponding hydrochloride **(39).**

**7.** A method for producing compounds of the general formula **I** and the pharmaceutically acceptable salts thereof, **characterised in that** cycloalkanones of the general formula **II**

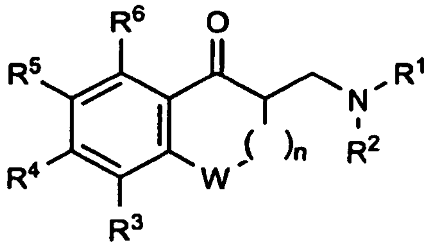

II

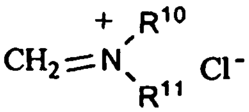

III

$NHR^{10}R^{11}$

IV

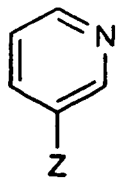

V

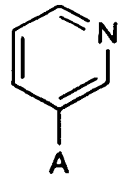

VI

are reacted with immonium salts of the formula **III** or with paraformaldehyde and an amine of the formula **IV,** $R^{10}$ having a meaning similar to $R^1$ and $R^{11}$ a meaning similar to $R^2$, the resultant Mannich bases are reacted in solvent between -70°C and 60°C with an organometallic compound of the formula **V,** in which Z means Li, it being the case that if either $R^{10}$ or $R^{11}$ or both simultaneously are hydrogen, compounds of the general formula **III** or **IV,** in which $R^{10}$ or $R^{11}$ or $R^{10}$ and $R^{11}$ represent(s) a benzyl residue, which is removed by catalytic reaction with hydrogen, are used in the Mannich reaction and compounds of the formula **V,** in which Z means lithium or magnesium halide, being produced by halogen/lithium exchange by reacting halogen compounds of the formula **VI,** in which A means Cl, Br or I, with preferably n-butyllithium/hexane solution, or compounds of the formula **V** being reacted in the presence of cerium(II) halide with compounds of the formula **II,** whereby products of the general formula **VII,**

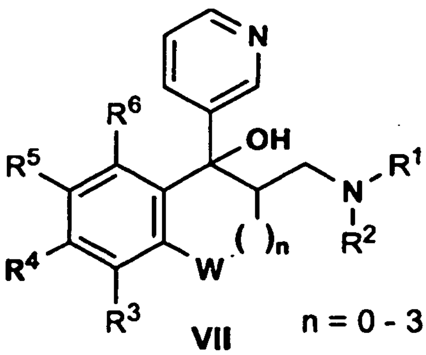

VII n = 0 - 3

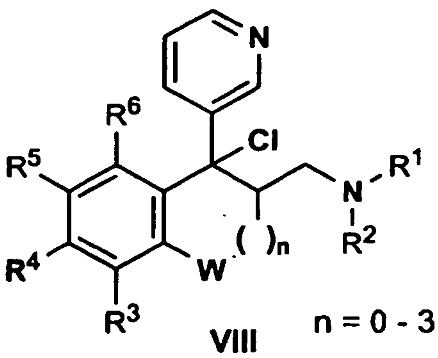

VIII n = 0 - 3

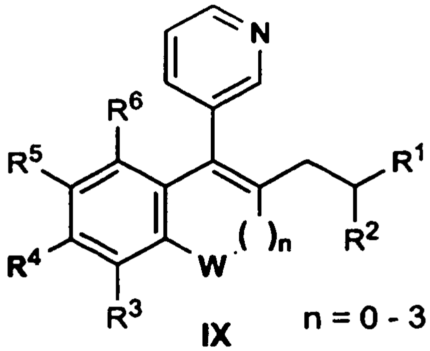

IX n = 0 - 3

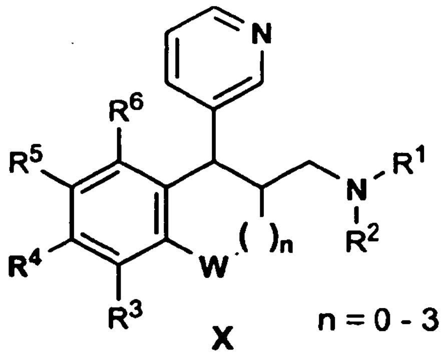

X n = 0 - 3

in which $R^{10}$ has a meaning similar to $R^1$ and $R^{11}$ a meaning similar to $R^2$, are obtained, which are reacted with thionyl chloride and then worked up under basic conditions to yield compounds of the general formula **IX** or a mixture of compounds of the general formulae **VIII** and **IX,** in which $R^{10}$ has a meaning similar to $R^1$ and $R^{11}$ a meaning similar to $R^2$, the compounds are separated, or the compounds of the general formula **IX** are produced by reacting compounds of the general formula **VII** with strong acids, then hydrogenolytic cleavage of compounds of the general formula **VIII** or hydrogenation of compounds of the general formula **IX,** in which $R^{10}$ has a meaning similar to $R^1$ and $R^{11}$ a meaning similar to $R^2$, by catalytic reaction with hydrogen to yield compounds of the formula **X,** in which $R^{10}$ has a meaning similar to a $R^1$ and $R^{11}$ a meaning similar to $R^2$, is carried out in solvent at pressures of 0.1 to 10 bar and temperatures of 20°C to 80°C, with compounds of the general formula **I** where W is SO or $SO_2$ being produced by oxidation from corresponding compounds where W is S.

**8.** A method for producing unsaturated 3-pyridyl benzocycloalkylmethylamines of the general formula **I,** in which X and Y together form a bond, **characterised in that** transition metal-catalysed cross-coupling of compounds of the general formula **XI,**

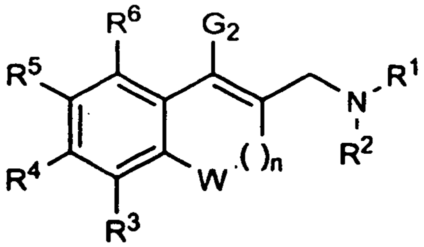

XI

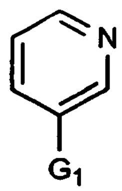

XII

in which $G_2$ means Cl, Br, I, $Sn(alkyl)_3$ or $OSO_2CF_3$ proceeds with compounds of the general formula **XII,** in which $G_1$ means Cl, Br, I or $B(OR^x)_2$, with $R^x$ selected from H or alkyl, it being possible to carry out the reaction with or without base and with or without ligand.

**9.** A method for producing unsaturated 3-pyridyl benzocycloalkylmethylamines of the general formula **I,** in which X and Y together form a bond and W is O or S and n is 1, **characterised in that** ortho-halo-substituted phenols, with halogen being selected from Br, I, $OSO_2CF_3$, thiophenols of the general formula **XIII**

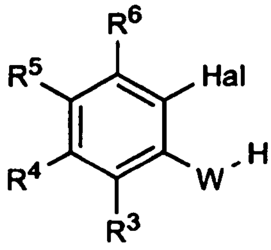
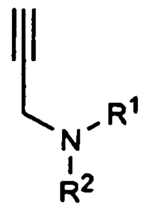
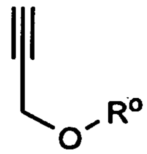

**XIII** **XIV** **XV**

are reacted with substituted propargylamines of the general formula **XIV** or with propargyl alcohols or the derivatives thereof of the general formula **XV** with R° selected from H or a protective group, with, in the case of propargyl alcohols or the derivatives thereof of the general formula **XV,** the function OR° being converted in a manner known per se in a downstream reaction step into the amino function $NR^1R^2$.

**10.** A method according to any one of claims 7, 8 or 9, **characterised in that** at least one OH group present in formula **I** is replaced by an $OSi(Ph)_2$tert.but. group, at least one SH-group by an S-p-methoxybenzyl group and/or at least one $NH_2$ group by an $NO_2$ group and, after completion of the entire reaction sequence, an $OSi(Ph)_2$tert.but. group is eliminated with tetrabutylammonium fluoride in tetrahydrofuran and/or at least one p-methoxybenzyl group with a metal amine, preferably sodium amine, and/or at least one $NO_2$ group is reduced to $NH_2$.

**11.** A medicament containing at least one compound of the general formula **I** or one of the pharmaceutically acceptable salts thereof.

**12.** Use of a compound of the general formula **I** or one of the pharmaceutically acceptable salts thereof for producing a medicament for treating pain, depression and/or anxiety disorders.

**13.** Use of a compound of the general formula **I** or of one of the pharmaceutically acceptable salts thereof for producing a medicament for treating migraine, urinary incontinence, fibromyalgia, eating disorders, bulimia, hyperactivity, drug dependency, addiction and withdrawal, trichotillomania, Tourette's syndrome, skin conditions such as post-herpetic neuralgia and pruritus, psychoses, memory disorders, cognitive disorders and/or Alzheimer's disease.

**14.** A pharmaceutical composition which contains at least one compound of the general formula **I** or one of the pharmaceutically acceptable salts thereof together with at least one pharmaceutical auxiliary substance.

**Revendications**

**1.** 3-pyridylbenzocycloalkylméthylamines saturées et insaturées de formule générale I,

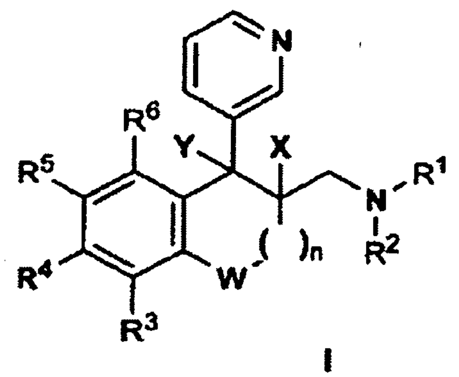

**I**

où

W représente $CH_2$, O, S, SO ou $SO_2$ et n = 0-3,
$R^1$ et $R^2$ sont choisis, indépendamment l'un de l'autre, parmi H et $C_1$-$C_{10}$-alkyle, à chaque fois ramifié ou non ramifié, monosubstitué ou polysubstitué ou non substitué ;
et
$R^3$ à $R^6$ sont choisis, indépendamment l'un de l'autre parmi H ou des radicaux quelconques, à l'exception d'autres cycles condensés, où on entend par ces radicaux F, Cl, Br, I, CN, $CF_3$, $OCF_3$, $C_1$-$C_{10}$-alkyle, à chaque fois ramifié ou non ramifié, monosubstitué, polysubstitué ou non substitué ;
$OR^{6'}$ ou $SR^{6'}$ où $R^{6'}$ est choisi parmi H et $C_1$-$C_{10}$-alkyle, à chaque fois ramifié ou non ramifié, monosubstitué ou polysubstitué ou non substitué ;
et Y est choisi parmi H ou OH, lorsque X représente simultanément H, ou X et Y forment ensemble une liaison,

où on entend par "substitué" la substitution d'un radical hydrogène par F, Cl, Br, I, $NH_2$, SH, OH ou $OCH_3$, également sous forme de leurs racémates, énantiomères, diastéréo-isomères, en particulier des mélanges de leurs énantiomères ou diastéréo-isomères ou d'un seul énantiomère ou diastéréo-isomère ainsi que leurs bases libres ou un sel formé avec un acide physiologiquement acceptable.

**2.** Composés de formule générale I selon la revendication 1, **caractérisés en ce que** n < 3.

**3.** Composés de formule générale I selon la revendication 1 ou 2, **caractérisés en ce que** W = $CH_2$ et n < 3.

**4.** Composés de formule générale I selon l'une quelconque des revendications 1 à 3, **caractérisés en ce que** W = $CH_2$, n < 3, $R^1$ et $R^2$ = méthyle et $R^5$ et $R^6$ = H.

**5.** Composés de formule générale I selon la revendication 1 ou 2, où les composés sont choisis parmi :

2-diméthylaminométhyl-1-pyridin-3-yl-indan-1-ol et le chlorhydrate correspondant (diastéréo-isomère polaire) (1)
2-diméthylaminométhyl-1-pyridin-3-yl-indan-1-ol et le chlorhydrate correspondant (diastéréo-isomère non polaire) (2)
diméthyl-(3-pyridin-3-yl-1H-indén-2-ylméthyl)-amine et le chlorhydrate correspondant (3)
diméthyl-(1-pyridin-3-yl-indan-2-ylméthyl)-amine et le chlorhydrate correspondant (4)
2-diméthylaminométhyl-5-méthoxy-1-pyridin-3-yl-indan-1-ol et le chlorhydrate correspondant (diastéréo-isomère polaire et non polaire) (5)
(6-méthoxy-3-pyridin-3-yl-1H-indén-2-ylméthyl)-diméthylamine et le chlorhydrate correspondant (6)
2-diméthylaminométhyl-1-pyridin-3-yl-3H-indén-5-ol et le chlorhydrate correspondant (7)
2-diméthylaminométhyl-1-pyridin-3-yl-1,2,3,4-tétrahydronaphtalén-1-ol et le chlorhydrate correspondant (diastéréo-isomère polaire et non polaire) (8)
diméthyl-(1-pyridin-3-yl-3,4-dihydronaphtalén-2-ylméthyl)-amine et le chlorhydrate correspondant (9)
diméthyl-(1-pyridin-3-yl-1,2,3,4-tétrahydronaphtalén-2-ylméthyl)-amine et le chlorhydrate correspondant (10)
6-diméthylaminométhyl-5-pyridin-3-yl-6,7,8,9-tétrahydro-5H-benzocycloheptén-5-ol et le chlorhydrate correspondant (11)
diméthyl-(5-pyridin-3-yl-8,9-dihydro-7H-benzocycloheptén-6-ylméthyl)-amine et le chlorhydrate correspondant (12)
4-diméthylaminométhyl-5-pyridin-3-yl-2,3,4,5-tétrahydrobenzo[b]thiépin-5-ol et le chlorhydrate correspondant (diastéréo-isomère polaire) (13)
4-diméthylaminométhyl-5-pyridin-3-yl-2,3,4,5-tétrahydrobenzo[b)thiépin-5-ol et le chlorhydrate correspondant (diastéréo-isomère non polaire) (14)
diméthyl-(5-pyridin-3-yl-2,3-dihydrobenzo[b]thiépin-4-ylméthyl)-amine et le chlorhydrate correspondant (15)
diméthyl-(1-oxo-5-pyridin-3-yl-2,3-dihydro-1H-1λ4-benzo[b)thiépin-4-ylméthyl)-amine et le chlorhydrate correspondant (16)
4-diméthylaminométhyl-1,1-dioxo-5-pyridin-3-yl-2,3,4,5-tétrahydro-1H-1λ6-benzo[b]thiépin-5-ol et le chlorhydrate correspondant (17)
2-diméthylaminométhyl-5-fluoro-1-pyridin-3-yl-indan-1-ol et le chlorhydrate correspondant (diastéréo-isomère polaire et non polaire) (18)
(6-fluoro-3-pyridin-3-yl-1H-indén-2-ylméthyl)-diméthylamine et le chlorhydrate correspondant (19)
2-diméthylaminométhyl-1-pyridin-3-yl-5-trifluorométhoxyindan-1-ol et le chlorhydrate correspondant (diastéréo-isomère polaire et non polaire) (20)
diméthyl-(3-pyridin-3-yl-6-trifluorométhoxy-1H-indén-2-ylméthyl)-amine et le chlorhydrate correspondant (21)
2-diméthylaminométhyl-1-pyridin-3-yl-5-trifluorométhylindan-1-ol et le chlorhydrate correspondant (diastéréo-isomère polaire et non polaire) (22)
diméthyl-(3-pyridin-3-yl-6-trifluorométhyl-1H-indén-2-ylméthyl)-amine et le chlorhydrate correspondant (23)
4-diméthylaminométhyl-5-pyridin-3-yl-2,3,4,5-tétrahydrobenzo[b]oxépin-5-ol et le chlorhydrate correspondant (diastéréo-isomère non polaire) (24)
4-diméthylaminométhyl-5-pyridin-3-yl-2,3,4,5-tétrahydrobenzo[b]oxépin-5-ol et le chlorhydrate correspondant (diastéréo-isomère polaire) (25)
diméthyl-(5-pyridin-3-yl-2,3-dihydrobenzo[b]oxépin-4-ylméthyl)-amine et le chlorhydrate correspondant (26)
3-diméthylaminométhyl-4-pyridin-3-yl-chroman-4-ol et le chlorhydrate correspondant (diastéréo-isomère non polaire) (27)
3-diméthylaminométhyl-4-pyridin-3-yl-chroman-4-ol et le chlorhydrate correspondant (diastéréo-isomère polaire) (28)
diméthyl-(4-pyridin-3-yl-2H-chromén-3-ylméthyl)-amine et le chlorhydrate correspondant (29)
3-diméthylaminométhyl-4-pyridin-3-yl-thiochroman-4-ol et le chlorhydrate correspondant (diastéréo-isomère non polaire) (30)
3-diméthylaminométhyl-4-pyridin-3-yl-thiochroman-4-ol et le chlorhydrate correspondant (diastéréo-isomère polaire) (31)
diméthyl-(4-pyridin-3-yl-2H-thiochromén-3-ylméthyl)-amine et le chlorhydrate correspondant (32)
2-diméthylaminométhyl-6-méthoxy-1-pyridin-3-yl-indan-1-ol et le chlorhydrate correspondant (diastéréo-isomère non polaire) (33)
2-diméthylaminométhyl-6-méthoxy-1-pyridin-3-ylindan-1-ol et le chlorhydrate correspondant (diastéréo-isomère polaire) (34)
(5-méthoxy-3-pyridin-3-yl-1H-indén-2-ylméthyl)-diméthylamine et le chlorhydrate correspondant (35)
2-diméthylaminométhyl-5-méthoxy-1-pyridin-3-yl-1,2,3,4-tétrahydronaphtalén-1-ol et le chlorhydrate correspondant (diastéréo-isomère non polaire) (36)
2-diméthylaminométhyl-5-méthoxy-1-pyridin-3-yl-1,2,3,4-tétrahydronaphtalén-1-ol et le chlorhydrate correspondant (diastéréo-isomère polaire) (37
(5-méthoxy-1-pyridin-3-yl-3,4-dihydronaphtalén-2-ylméthyl)-diméthylamine et le chlorhydrate correspondant (38)
6-diméthylaminométhyl-5-pyridin-3-yl-7,8-dihydronaphtalén-1-ol et le chlorhydrate correspondant (39).

**6.** Composés selon la revendication 1, 2 ou 5,
**caractérisés en ce qu'**ils sont choisis parmi :

diméthyl-(3-pyridin-3-yl-1H-indén-2-ylméthyl)-amine et le chlorhydrate correspondant (3)
(6-méthoxy-3-pyridin-3-yl-1H-indén-2-ylméthyl)-diméthylamine et le chlorhydrate correspondant (6) 2-diméthylaminométhyl-1-pyridin-3-yl-3H-indén-5-ol et
le chlorhydrate correspondant (7) 6-diméthylaminométhyl-5-pyridin-3-yl-6,7,8,9-tétrahydro-5H-benzocycloheptén-5-ol et le chlorhydrate correspondant (11)
diméthyl-(5-pyridin-3-yl-8,9-dihydro-7H-benzocycloheptén-6-ylméthyl)-amine et le chlorhydrate correspondant (12)
(6-fluoro-3-pyridin-3-yl-1H-indén-2-ylméthyl)-diméthylamine et le chlorhydrate correspondant (19)
diméthyl-(3-pyridin-3-yl-6-trifluorométhoxy-1H-indén-2-ylméthyl)-amine et le chlorhydrate correspondant (21)
(5-méthoxy-1-pyridin-3-yl-3,4-dihydronaphtalén-2-ylméthyl)-diméthylamine et le chlorhydrate correspondant (38)
6-diméthylaminométhyl-5-pyridin-3-yl-7,8-dihydronaphtalén-1-ol et le chlorhydrate correspondant (39).

**7.** Procédé pour la préparation de composés de formule générale I ainsi que leurs sels pharmaceutiquement acceptables, **caractérisé en ce qu'**on transforme des cycloalcanones de formule générale II

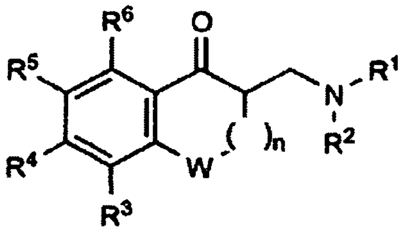

II

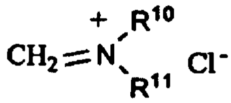

$NHR^{10}R^{11}$

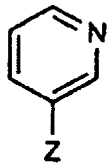

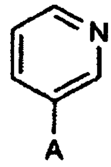

III IV V VI

avec des sels d'immonium de formule III ou avec du paraformaldéhyde et une amine de formule IV, où $R^{10}$ a une signification analogue à $R^1$ et $R^{11}$ a une signification analogue à $R^2$, on transforme les bases de Mannich obtenues avec un composé métallo-organique de formule V, dans laquelle Z signifie Li, dans des solvants entre -70°C et 60°C, où, dans le cas où soit $R^{10}$ soit $R^{11}$ soit les deux simultanément représentent hydrogène, on utilise dans la réaction de Mannich des composés de formule générale III ou IV, dans laquelle $R^{10}$ ou $R^{11}$ ou $R^{10}$ et $R^{11}$ représente (nt) un radical benzyle, qui est éliminé par transformation catalytique avec de l'hydrogène et où on prépare des composés de formule V, dans laquelle Z signifie lithium ou halogénure de magnésium, par transformation de composés halogénés de formule VI, dans laquelle A signifie Cl, Br ou I, avec de préférence une solution de n-butyllithium/hexane par remplacement halogène-lithium, ou on transforme des composés de formule V en présence d'un halogénure de cérium (II) avec des composés de formule II, suite à quoi on obtient des produits de formule générale VII,

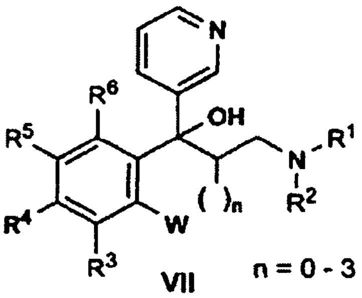

VII n = 0 - 3

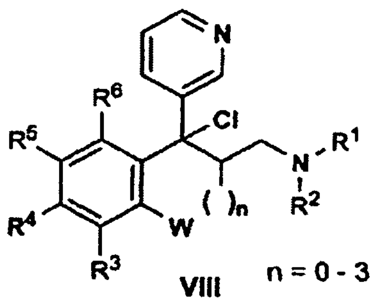

VIII n = 0 - 3

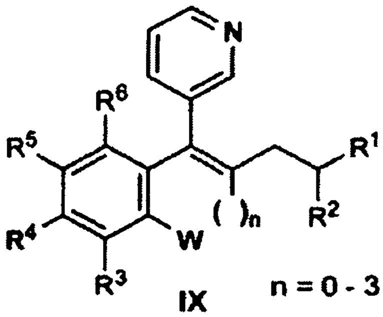

IX n = 0 - 3

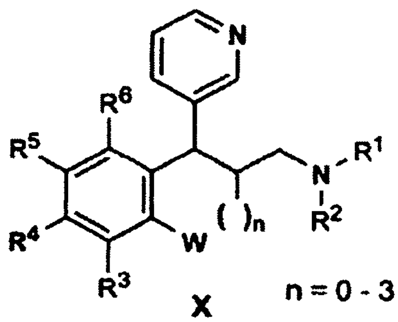

X n = 0 - 3

dans laquelle $R^{10}$ a une signification analogue à $R^1$ et $R^{11}$ a une signification analogue à $R^2$, qui sont transformés

avec du chlorure de thionyle et ensuite traités de manière basique en composés de formule générale IX ou un mélange de composés de formule générale VIII et IX, dans lesquelles $R^{10}$ a une signification analogue à $R^1$ et $R^{11}$ a une signification analogue à $R^2$, on sépare les composés ou on prépare des composés de formule générale IX par transformation de composés de formule générale VII avec des acides forts, puis on réalise la dissociation par hydrogénolyse de composés de formule générale VIII ou l'hydrogénation de composés de formule générale IX, dans laquelle $R^{10}$ a une signification analogue à $R^1$ et $R^{11}$ a une signification analogue à $R^2$, par réaction catalytique avec de l'hydrogène en composés de formule X, dans laquelle $R^{10}$ a une signification analogue à $R^1$ et $R^{11}$ a une signification analogue à $R^2$, dans des solvants à des pressions de 0,1 à 10 bars et des températures de 20°C à 80°C, où on prépare des composés de formule générale I avec W représentant SO ou $SO_2$ à partir de composés correspondants avec W représentant S par oxydation.

8. Procédé pour la préparation de 3-pyridylbenzocycloalkylméthylamines insaturées de formule générale I, dans laquelle X et Y forment ensemble une liaison, **caractérisé en ce qu'**on réalise un couplage croisé catalysé par un métal de transition de composés de formule générale XI,

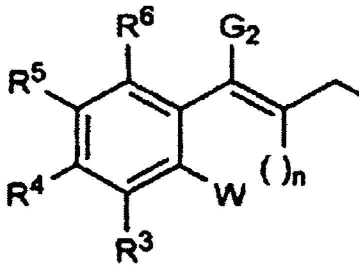

XI

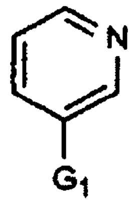

XII

dans laquelle $G_2$ représente Cl, Br, I, Sn(alkyle)$_3$ ou $OSO_2CF_3$, avec des composés de formule générale XII, dans laquelle $G_1$ signifie Cl, Br, I ou B(OR$^x$)2, avec $R^x$ choisi parmi H ou alkyle, où la réaction peut être réalisée avec ou sans base et avec ou sans ligand.

9. Procédé pour la préparation de 3-pyridylbenzocycloalkylméthylamines insaturées de formule générale I, dans laquelle X et Y forment ensemble une liaison et W représente O ou S et n = 1, **caractérisé en ce qu'**on transforme des phénols substitués par halogéno en position ortho, où halogène est choisi parmi Br, I, $OSO_2CF_3$, /des thiophénols de formule générale XIII

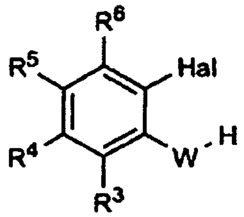

XIII

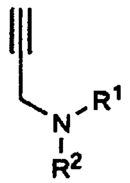

XIV

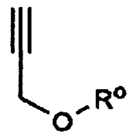

XV

avec des propargylamines substituées de formule générale XIV ou avec des alcools propargyliques ou leurs dérivés de formule générale XV avec R° choisi parmi H ou un groupe de protection, où, dans le cas des alcools propargyliques ou leurs dérivés de formule générale XV, la fonction OR° est transformée dans une étape de réaction consécutive de manière connue en soi en fonction amino $NR^1R^2$.

10. Procédé selon l'une quelconque des revendications 7, 8 ou 9, **caractérisé en ce qu'**au moins un groupe OH contenu dans la formule I est remplacé par un groupe OSi(Ph)$_2$tert-butyle, au moins un groupe SH est remplacé par un groupe S-p-méthoxybenzyle et/ou au moins un groupe $NH_2$ est remplacé par un groupe $NO_2$ et après la fin de la séquence globale de réaction, un groupe OSi(Ph)$_2$tert-butyle est dissocié avec du fluorure de tétrabutylammonium

dans du tétrahydrofuranne et/ou au moins un groupe p-méthoxybenzyle est dissocié avec une amine métallique, de préférence une amine sodique et/ou au moins un groupe $NO_2$ est réduit en $NH_2$.

**11.** Médicament, contenant au moins un composé de formule générale I ou un de ses sels pharmaceutiquement acceptables.

**12.** Utilisation d'un composé de formule générale I ou d'un de ses sels pharmaceutiquement acceptables pour la préparation d'un médicament destiné au traitement de douleurs, de dépressions et/ou de troubles d'anxiété.

**13.** Utilisation d'un composé de formule générale I ou d'un de ses sels pharmaceutiquement acceptables pour la préparation d'un médicament destiné au traitement de la migraine, de l'incontinence urinaire, de la fibromyalgie, de troubles alimentaires, de la boulimie, de l'hyperactivité, de la dépendance de médicaments, de la toxicomanie et du sevrage de médicaments, de la trichotillomanie, du syndrome de Gilles de la Tourette, de maladies de la peau, telles que la neuralgie post-herpétique et du prurit, de psychoses, de troubles de la mémoire, de troubles cognitifs et/ou de la maladie d'Alzheimer.

**14.** Composition pharmaceutique, qui contient au moins un composé de formule générale I ou un de ses sels pharmaceutiquement acceptables ainsi qu'au moins un adjuvant pharmaceutique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 2004149429 A
- WO 9958490 A
- US 6159996 A1


### In der Beschreibung aufgeführte Nicht-Patentliteratur


- **Sindrup et al.** Anesthesia. Biological foundations. Lippincott-Raven, 1997, 987-997
- **Pacher, P. ; Kohegyi, E. ; Kecskemeti, V. ; Furst, S.** *Current Medicinal Chemistry,* 2001, vol. 8, 89-100
- **Goddard, A. W. ; Coplan, J.D. ; Gorman, J. M. ; Chamey, D. S.** Neurobiology of mental illness. Oxford University Press, 1999, 548-563
- **A. Geronikaki et al.** *Pharmazie,* 1989, vol. 44, 349
- Remington's Pharmaceutical Sciences. Mack Publishing Company, 1985
- **A. Orita ; J. Yaruva ; J. Otera.** *Angew. Chem.,* 1999, vol. 111, 2397
- **Gray, E.G. ; Whittaker, V.P.** *J. Anat.,* 1962, vol. 76, 79-88
- **Frink, M. Ch. ; Hennies, H.H. ; Englberger, W. et al.** *Arzneim.-Forsch./Drug Res.,* 1996, vol. 46 (III), 11, 1029-1036
- **Cheng, Y.C. ; Prusoff, W.H.** *Biochem. Pharmacol.,* 1973, vol. 22, 3099-3108
- **Lineweaver, H. ; Burk, D.** *J. Am. Chem. Soc.,* 1934, vol. 56, 658-666
- **Dubuisson, D. ; Dennis, S.G.** *Pain,* 1977, vol. 4, 161-174